# FASCICULE DE BREVET EUROPEEN

(11) **EP 0 354 072 B1**
(45) Date de publication et mention de la délivrance du brevet: **10.05.1995**
(21) Numéro de dépôt: 89401617.9
(22) Date de dépôt: 09.06.1989
(51) Int. Cl.: A61K 39/21, A61K 39/395, C12P 21/00, C07K 14/155, C07K 1/00

(54) **Précurseur de la glycoprotéine d'enveloppe du rétrovirus HIV-2 et antigènes apparentés**
Vorläufer des Hüllenglykoproteins des HIV-2 Retrovirus und verwandte Antigene
Precursors of the HIV-2 retrovirus envelope glycoprotein and related antigens

(30) Priorité: 09.06.1988 US 204346; 10.06.1988 FR 8807817
(43) Date de publication de la demande: 07.02.1990
(73) Titulaire: INSTITUT PASTEUR, 75724 Paris Cédex 15 (FR)
(72) Inventeur: Montagnier, Luc, F-92350 Le Plessis-Robinson (FR); Hovanessian, Ara, F-93100 Montreuil (FR); Laurent, Anne, F-75007 Paris (FR); Krust, Bernard, F-75012 Paris (FR); Rey, Marie-Anne, F-75004 Paris (FR)
(74) Mandataire: Gutmann, Ernest

(56) Documents cités:
- EP-A- 0 269 520
- WO-A-87/02892
- WO-A-88/05440
- BIOLOGICAL ABSTRACTS, vol. 87; B. KRUST et al., no. 110761/
- CHEMICAL ABSTRACTS, vol. 110, no. 13, 27 mars 1989, Columbus, OH (US); M.A. REY et al., p. 385, no. 111412v/
- PROCEEDINGS OF THE NATL. ACADEMY OF SCIENCES USA, vol. 84, novembre 1987, Washington, DC (US); B.D. WALKER et al., pp. 8120-8124/

## Description

L'invention est relative à des antigènes précurseurs des glycoprotéines d'enveloppe d'un rétrovirus humain HIV-2 et à des antigènes croisant immunologiquement avec des anticorps monoclonaux spécifiques reconnaissant les antigènes ci-dessus, précurseurs des glycoprotéines d'enveloppe du rétrovirus HIV-2.

L'invention concerne plus précisément des antigènes présents exclusivement lors de certaines phases de l'évolution in vivo d'une infection due à un rétrovirus humain HIV-2. Ces antigènes sont caractéristiques du processus de formation et de maturation de la glycoprotéine d'enveloppe de ce rétrovirus, et sont en conséquence impliqués dans le développement et la prolifération du rétrovirus HIV-2 et partant dans la propagation de l'infection.

L'invention a également trait à des anticorps reconnaissant ces antigènes, ainsi qu'à des compositions immunogènes contenant ces anticorps.

L'invention concerne en outre la préparation des antigènes précédents, ainsi que leur utilisation pour le diagnostic d'une infection par un rétrovirus humain HIV-2.

Des agents étiologiques responsables du développement de syndrômes de lymphadénopathies (dont l'abréviation anglaise est SLA) ou concourant au développement de ces syndrômes ou responsables du développement de syndrômes d'immunodéficience acquise (SIDA) ont été isolés, identifiés et caractérisés.

On a désigné par HIV (abréviation anglaise de Human Immunodéficiency Virus) plusieurs rétrovirus humains susceptibles, dans certaines conditions, de provoquer un SLA éventuellement relayé par un SIDA chez l'homme.

Ainsi un premier virus dénommé LAV-1 ou HIV-1 a été isolé et décrit dans la demande de brevet GB.83/24.800 correspondant à la demande EP.84/401.834 du 14/09/84 publiée sous le numéro 0 138 667. Ce virus a également été décrit par F.Barre Sinoussi et al. dans Science, 220 n° 45-99, 20, pages 868-871.

Des variants de ce virus HIV-1 désignés par LAV ELI et LAV MAL, ont également été isolés, caractérisés et décrits dans la demande de brevet EP.84/401.834.

L'isolement et la caractérisation de rétrovirus appartenant à une classe distincte et n'ayant qu'une parenté immunologique réduite avec les précédents, ont été décrits dans la demande de brevet européen n° 87/400.151.4. publiée sous le n° 239.425. Ces rétrovirus qui ont été regroupés sous la désignation HIV-2, ont été isolés chez plusieurs malades africains présentant des symptômes d'une lymphadénopathie ou d'un SIDA.

L'étude de ces rétrovirus HIV l'isolement et l'analyse de leurs séquences nucléotidiques, ainsi que la caractérisation de leurs protéines antigéniques a permis d'effectuer des études de comparaison entre les différentes souches obtenues au niveau de leur parenté ou au contraire de leur spécicité tant structurales que fonctionnelles.

Ces rétrovirus HIV-1 et HIV-2 ont également été étudiés en comparaison avec un rétrovirus d'origine simienne (désigné par l'abréviation SIV ou STLV-III) et cette étude a permis de montrer une certaine parenté entre les protéines et glycoprotéines du rétrovirus HIV-2 et de ses variants et celles du rétrovirus SIV. Une parenté est notamment remarquée au niveau de la protéine d'enveloppe de chacun de ces virus. On a en effet mis en évidence des réactions immunologiques croisées entre la glycoprotéine d'enveloppe du rétrovirus SIV et des anticorps dirigés contre la glycoprotéine d'enveloppe du rétrovirus HIV-2, alors que cette réaction de croisement immunologique ne se produit pas entre le rétrovirus SIV et le rétrovirus HIV-1 ni entre les rétrovirus HIV-1 et HIV-2.

Des résultats concernant la glycoprotéine d'enveloppe du rétrovirus HIV-2 codée par le gène env du génome de ce rétrovirus ont été donnés dans la demande de brevet européen n° 87/400.151.4. (publiée sous le numéro 0 239 425) précitée. Dans cette demande de brevet EP, cette glycoprotéine a été désignée par l'abréviation gp140 par référence à son poids moléculaire d'environ 140.000d. Il était précisé toutefois que le calcul du poids moléculaire était apprécié à ± 10%. Dans une demande de brevet France précédente (n° 86/03881 publiée sous le n° 2.596.063) un précurseur de la gp140 désigné par gp160 (poids moléculaire de 160Kd ± 10%) avait également été décrit.

Des investigations plus poussées relatives à la glycoprotéine d'enveloppe du rétrovirus HIV-2 ont été conduites et ont permis aux inventeurs de réaliser d'autres mesures des poids moléculaires donnés jusqu'à présent et en conséquence d'affiner les résultats, à partir des intervalles de poids moléculaires décrits ci-dessus. La glycoprotéine externe d'enveloppe présente, dans les particules virales de HIV-2 - dans les conditions opératoires de la présente invention - un PM d'environ 125000d (elle sera désignée par gp125). Dans ces mêmes conditions opératoires, le précurseur déjà mis en évidence et désigné par gp160 dans la demande de brevet FR 86/03881 a, selon les derniers résultats obtenus, un poids moléculaire d'environ 140000d. Il sera donc mentionné par "gp140" dans la présente demande de brevet.

Dans le cadre de l'invention, les inventeurs ont mis en évidence l'existence de plusieurs phases de développement conduisant à l'obtention de glycoprotéines d'enveloppe sous une forme mature, ces glycoprotéines d'enveloppe matures comprenant une glycoprotéine gp125 et une glycoprotéine gp36. Les inventeurs ont donc identifié différents stades dans le déroulement du cycle de réplication virale apparaissant lorsque des troubles pathogènes se développent à la suite d'une infection par un rétrovirus humain HIV-2. Les inventeurs ont souligné à ce propos l'importance de plusieurs étapes se déroulant dans les cellules infectées par le rétrovirus HIV-2 notamment dans les lymphocytes T4 humains.

En parallèle, ils ont conduit des études similaires sur les cycles viraux des rétrovirus HIV-1 et SIV. Ces dernières études ont révélé des propriétés très spécifiques communes aux rétrovirus HIV-2 et SIV et au contraire apparemment inexistantes lors du cycle de réplication virale de HIV-1.

L'invention fournit donc de nouveaux moyens pour détecter une infection spécifiquement attribuable à un rétrovirus humain HIV-2.

Au vu des derniers résultats obtenus, le cycle viral propre aux rétrovirus HIV-2 et SIV apparaît comme un processus complexe de réactions en chaîne dont l'ensemble seul pourrait conduire à la formation de la glycoprotéine externe présente dans l'enveloppe du rétrovirus HIV-2 sous sa forme définitive. Lors de ce processus, différents précurseurs antigéniques ont pu être reconnus, isolés et identifiés. Tous possèdent notamment une parenté structurale fondée sur l'ossature peptidique de la glycoprotéine d'enveloppe codée par le gène env du rétrovirus HIV-2 décrit dans la demande de brevet EP. précitée.

L'invention est donc relative à des protéines ou à des glycoprotéines produites à la suite d'une infection par un rétrovirus humain HIV-2 lors des différentes étapes du cycle de formation et de maturation de la glycoprotéine externe d'enveloppe gp125 de HIV-2, ayant en commun l'ossature peptidique de la glycoprotéine externe d'enveloppe du rétrovirus humain HIV-2 ou d'une glycoprotéine présentant une réaction immunologique croisée avec la susdite gp125 et diffèrant au niveau de la présence et/ou de la composition des chaînes d'oligosaccharides éventuelles.

Les caractéristiques spécifiques des protéines et glycoprotéines de l'invention sont acquises lors de chaque étape indispensable à la formation et à la maturation de la glycoprotéine externe d'enveloppe mature du rétrovirus humain HIV-2.

Grâce à l'étude du cycle viral, les inventeurs ont suggéré la possibilité d'intervenir lors d'étapes particulières de la formation des glycoprotéines d'enveloppe mature du rétrovirus humain HIV-2, donnant ainsi des moyens pour interrompre le déroulement normal du cycle viral et remédier à la propagation du rétrovirus infectieux pour l'homme et pathogène dans certaines conditions.

En accord avec ce qui précède, l'invention concerne une glycoprotéine rétrovirale antigénique caractérisée en ce qu'il s'agit :
- soit d'une glycoprotéine, appelée gp300, ayant un poids moléculaire (PM) d'environ 300Kd, et constituée par l'association de deux unités d'une glycoprotéine gp140 précurseur de la glycoprotéine d'enveloppe gp125 codée par le gène env du génome d'un rétrovirus humain du type HIV-2 susceptible de provoquer un SLA ou SIDA,
- soit du dimère du précurseur de la glycoprotéine d'enveloppe d'un rétrovirus qui croise immunologiquement avec des anticorps dirigés contre ladite glycoprotéine gp300.

Il est remarqué que le précurseur gp140 est également le précurseur de la gp36 d'un rétrovirus humain de type HIV-2. Les inventeurs ont constaté que l'association précitée des deux unités de gp140 est une propriété intrinsèque de leur ossature peptidique.

La protéine antigénique gp300 est encore caractérisée par les propriétés suivantes :
- elle est présente dans les cellules d'un échantillon biologique d'un malade infecté par un rétrovirus de la famille de HIV-2, notamment dans les lymphocytes T4 humains ou dans des lignées permanentes dérivées de ces lymphocytes T4,
- son point isoélectrique (pI) est obtenu pour un pH appartenant à l'intervalle [6,8 - 7,8],
- elle est glycosylée par des chaînes oligosaccharidiques liées à l'azote,
- elle est stable dans les détergents ioniques, notamment le SDS 1%, dans les détergents non-ioniques, notamment le Triton®X-100 2%, dans l'urée 4M, dans les sels forts et les agents réducteurs,
- elle se dissocie spontanément in vitro en deux glycoprotéines d'un poids moléculaire d'environ 140Kd entre pH 4-5,
- après isolement et purification sur gel d'électrophorèse, elle se dissocie dans un tampon acétate in vitro en deux glycoprotéines d'un poids moléculaire d'environ 140Kd pour des valeurs du pH égales ou inférieures à 6,
- elle présente une bande d'électrophorèse sur gel de polyacrylamide SDS 7,5% en présence d'urée 6M, correspondant à un poids moléculaire de 300Kd après purification sur colonne d'immunoaffinité du type HIV-2 sérum-Sépharose® contenant des anticorps spécifiques obtenus à partir du sérum d'un patient infecté par le rétrovirus humain du type HIV-2.

Les inventeurs ont remarqué que la formation du dimère consistant en la gp300 est une étape transitoire du cycle viral et vraisemblablement nécessaire à la maturation des glycoprotéines d'enveloppe du rétrovirus humain du type HIV-2. En effet, la protéine antigénique gp300 ci-dessus est caractérisée en ce qu'elle n'est pas révélée par analyse en électrophorèse d'un milieu biologique constitué de particules virales présentes dans le milieu de culture spécifique des cellules infectées par un rétrovirus humain du type HIV-2 après marquage avec de la ³⁵S-méthionine (200»Ci/ml ; 4 x 10⁶ cellules /ml) pendant 18 heures et purification des extraits obtenus, contenant les cellules infectées et leur milieu de culture correspondant, alors qu'elle est révélée dans les mêmes conditions dans les susdites cellules infectées.

Ceci les a amenés à considérer la gp300 comme un précurseur transitoire spécifique des glycoprotéines d'enveloppe finales. Cette propriété du cycle viral d'un rétrovirus humain du type HIV-2 résultant dans l'obtention d'une protéine gp300, est originale et inhabituelle par rapport aux connaissances acquises sur les mécanismes généraux de formation de glycoprotéines contenant des chaînes de glycosylation oligosaccharidiques liées sur l'azote.

Par ailleurs, les inventeurs ont remarqué de façon tout à fait intéressante que la formation et la maturation des glycoprotéines d'enveloppe du rétrovirus SIV_{mac} fait également intervenir une étape de dimérisation conduisant à l'obtention d'une glycoprotéine du type de la gp300 précisément caractérisée pour un rétrovirus humain de type HIV-2. Au contraire, l'observation parallèle du cycle viral du rétrovirus humain HIV-1 n'a permis de constater aucune étape de dimérisation résultant dans la formation d'une glycoprotéine apparentée à la gp300.

Ces résultats indiquent de façon tout à fait surprenante que la formation d'un doublet (ou dimère) à partir du précurseur gp140 de la glycoprotéine de l'enveloppe d'un rétrovirus humain du type HIV-2 est une propriété spécifique de l'expression du gène env des rétrovirus humains de type HIV-2 et SIV.

Cette constatation peut être mise à profit dans la recherche et le développement de moyens pour le diagnostic sélectif d'une infection due à un rétrovirus humain de type HIV-2 capable de provoquer un SLA ou un SIDA dans certaines conditions. On reviendra dans la suite sur ces moyens de diagnostic appropriés.

L'invention concerne également une protéine présentant des caractéristiques identiques à celles de la gp300 au niveau de l'ossature peptidique caractérisée en ce qu'elle est dépourvue de groupes de glycosylation et en ce que son poids moléculaire est d'environ 200Kd.

Cette protéine p200 sur laquelle on reviendra au cours des exemples qui suivent a été mise en évidence lors d'une expérience de digestion avec l'endo-β-N-acétylglucosaminidase H (désignée encore par "endo-H"). Cette protéine qui représente la forme non glycosylée de la gp300 pourrait avoir un intérêt particulier du fait de l'existence éventuelle d'un épitope commun à la gp300 et à la p200, qui s'avèrerait peu antigénique dans la gp300 du fait de la présence de chaînes oligosaccharidiques importantes pour la conformation finale de la gp300 mais néanmoins gênante pour l'accessibilité à un épitope intéressant.

Lors de l'étude du cycle viral, une autre glycoprotéine a été détectée ; il s'agit d'une glycoprotéine purifiée gp150, caractérisée en ce qu'elle est détectée in vitro (uniquement dans des cellules d'un patient infecté par un rétrovirus humain du type HIV-2) après marquage de ces cellules avec un traceur radioactif ajouté pendant une brève période, en présence d'un inhibiteur des enzymes glucosidases, notamment la castanospermine, puis élimination de ce traceur radioactif par lavage et remplacement par des molécules non radioactives préalablement à une étape de détermination de la radioactivité sur des échantillons prélevés à différents intervalles de temps, étape qui permet de répérer ce traceur dans les protéines et glycoprotéines formées après ledit marquage. L'observation de la gp150 est réalisée après un temps égal ou voisin de celui nécessaire à la détection de la gp300.

La glycoprotéine gp150 est encore caractérisée par :
- un poids moléculaire d'environ 150Kd,
- l'ossature peptidique du précurseur de la glycoprotéine d'enveloppe gp125, codée par le gène env du génome d'un rétrovirus humain du type HIV-2 ou d'un rétrovirus dont la glycoprotéine externe d'enveloppe est reconnue par les anticorps d'un sérum de patient infecté par ledit rétrovirus humain de type HIV-2, notamment les anticorps dirigés contre la gp125,
- des résidus glucose terminaux sur une chaîne d'oligosaccharides.

Les protéines et glycoprotéines précédemment décrites et de préférence la gp300, se sont avérées avantageuses pour l'utilisation dans des compositions antigéniques pour le diagnostic de la présence d'anticorps dans les cellules d'un échantillon biologique d'un patient infecté par un rétrovirus humain du type HIV-2, ces compositions étant caractérisées en ce qu'elles comprennent une protéine ou une glycoprotéine antigénique définie plus haut.

Ces compositions sont particulièrement intéressantes dans la mesure où elles permettent la formation de complexes immunologiques du type antigène-anticorps avec des anticorps du sérum d'un patient infecté par un rétrovirus humain du type HIV-2. En conséquence ces compositions et/ou les antigènes qu'elles contiennent seraient avantageusement mises en oeuvre pour effectuer le diagnostic spécifique d'une infection due à un rétrovirus humain du type HIV-2.

En outre, l'invention se rapporte à des anticorps monoclonaux caractérisés en ce qu'ils reconnaissent la gp300 définie dans les pages précédentes et en ce qu'ils ne reconnaissent pas la glycoprotéine d'enveloppe gp125 mature et la gp36 mature.

D'autres anticorps monoclonaux sont caractérisés en ce qu'ils reconnaissent un épitope qui, dans la gp300 ci-dessus, est exposé et accessible à l'anticorps, cet épitope étant, cependant, soit non-exposé, soit masqué dans les gp125 et gp36 matures dans leur conformation naturelle.

De tels anticorps monoclonaux peuvent aussi être neutralisants, notamment en empêchant la fixation d'un rétrovirus humain du type HIV-2 sur son récepteur, en particulier sur le récepteur T4 des lymphocytes humains qui le comportent.

D'autres anticorps monoclonaux intéressants dans le cadre de l'invention reconnaissent la p200 définie ci-dessus et ne reconnaissent pas les glycoprotéines d'enveloppe gp125 et gp36 matures.

D'autres anticorps monoclonaux sont caractérisés, en ce qu'ils reconnaissent un épitope dans la p200, cet épitope étant cependant soit non-exposé, soit masqué sur la gp125 et la gp36 et/ou sur la gp300 telles qu'obtenues dans leurs conformations naturelles.

Les anticorps monoclonaux ci-dessus peuvent avoir des propriétés immunogènes et neutralisantes, et interrompre la prolifération d'un rétrovirus humain de type HIV-2.

Plusieurs méthodes pour la production des anticorps précédents peuvent convenir dans le cadre de la réalisation de l'invention. Par exemple, on aura recours à des cultures en ascite chez les animaux, notamment chez les rongeurs comme le murin. On pourra également produire les anticorps à partir de cultures cellulaires soit immobilisées ou encapsulées ou encore en suspension. On utilisera notamment la technique des hybridomes obtenus à partir de la fusion de lymphocytes d'un animal auquel on aura préalablement inoculé un rétrovirus humain de type HIV-2. Des lymphocytes de l'animal infecté sont alors prélevés et fusionnés avec des cellules myelomateuses choisies, pour former des hybrides sélectionnés ensuite pour leur capacité à produire des anticorps spécifiques dirigés contre les protéines et glycoprotéines antigéniques décrites plus haut.

Ces anticorps monoclonaux produits seront par exemple utilisés pour inactiver les protéines et/ou glycoprotéines avec lesquelles ils forment un complexe immunologique ou encore pour leur capacité à empêcher le déroulement normal de la formation des glycoprotéines finales de l'enveloppe d'un rétrovirus humain de type HIV-2. De plus, étant donné la parenté de l'ossature peptidique des protéines et glycoprotéines de l'invention, les anticorps monoclonaux formés contre ces glycoprotéines, notamment contre la gp300, la p200 pourraient s'avérer efficaces contre la gp125.

Une autre application éventuelle de ces anticorps consisterait par exemple dans la détection d'antigènes viraux dans des préparations biologiques ou encore dans la purification de protéines et/ou glycoprotéines par exemple sur colonne d'affinité.

D'une façon générale, l'invention n'est pas limitée à des glycoprotéines et protéines telles que décrites précédemment lorsqu'elles sont obtenues à partir du seul rétrovirus humain du type HIV-2. Elle englobe au contraire des protéines et glycoprotéines présentant des propriétés antigéniques, immunologiques, voire immunogènes en commun avec les protéines et glycoprotéines ci-dessus décrites, ces protéines et glycoprotéines pouvant alors être définies par exemple par leur capacité à être reconnues par des anticorps dirigés spécifiquement contre les protéines correspondantes caractéristiques d'un rétrovirus humain du type HIV-2.

Cette remarque s'applique par exemple aux glycoprotéines gp300 et autres précurseurs de la glycoprotéine externe d'enveloppe d'un rétrovirus SIV_{mac} dont le cycle viral étudié par les inventeurs a montré des propriétés communes avec les protéines et glycoprotéines précitées d'un rétrovirus humain du type HIV-2.

L'invention est également relative à une composition immunogène contenant une glycoprotéine gp300 telle que définie ci-dessus en association avec un véhicule pharmaceutiquement acceptable approprié pour la préparation de vaccins.

Une composition immunogène ainsi formée est dosée en protéines et/ou glycoprotéines antigéniques de façon à permettre l'administration d'une dose de 10 à 100»g par kilogramme.

L'invention concerne encore un procédé de préparation de la glycoprotéine antigénique gp300 caractérisé par les étapes suivantes :
- lyse de cellules infectées par un rétrovirus humain du type HIV-2 et séparation du surnageant et de l'extrait cellulaire,
- incubation de l'extrait cellulaire et/ou du surnageant sur une colonne d'immunoaffinité, sur laquelle est déposé un immunoadsorbant renfermant des anticorps purifiés, tels qu'obtenus à partir du sérum d'un malade infecté par le rétrovirus humain d type HIV-2, fixés sur un support adapté, en présence d'un tampon et pendant un temps suffisamment long pour obtenir la formation d'un complexe immunologique antigène-anticorps,
- lavage de la colonne avec un tampon pour éliminer les molécules non retenues sur le support,
- résolution des protéines éluées, par exemple retenues par électrophorèse et par électroélution,
- récupération des protéines antigéniques recherchées.

Les cellules infectées auront pu être prélevées chez un patient infecté par un rétrovirus humain du type HIV-2. Elles auront également pu être obtenues in vitro à partir d'une culture de cellules infectées par HIV-2. De telles cultures cellulaires peuvent être réalisées sur des lignées CEM.

Un autre procédé pour la préparation de cette glycoprotéine est obtenu par analogie avec le précédent l'étape d'électroélution étant cependant remplacée par les étapes suivantes :
- élution des protéines fixées sur la colonne d'immunoaffinité ci-dessus,
- purification des produits ainsi élués sur une colonne de chromatographie contenant, fixés sur le support de séparation des anticorps monoclonaux dirigés contre la gp300,
   Dans un mode de réalisation particulier de l'invention la lyse des cellules infectées est réalisée dans une solution détergente, les anticorps compris dans l'immunoadsorbant sont fixés sur des billes d'agarose et on opère en présence d'un tampon d'accrochage.

L'invention vise également un procédé pour le diagnostic in vitro de la présence d'anticorps dans les cellules d'un échantillon biologique d'un patient, en vue de vérifier une infection éventuelle par un rétrovirus humain du type HIV-2, caractérisé par :
- la mise en contact de cet échantillon biologique avec un antigène décrit ci-dessus, ou une composition antigénique de l'invention, dans des conditions autorisant la formation d'un complexe immunologique du type antigène-anticorps.
- la détection du complexe antigène-anticorps forme. L'échantillon biologique sur lequel est effectué le diagnostic peut être un fluide biologique, notamment un sérum, un extrait de tissu biologique, dès lors qu'il est susceptible de contenir des cellules infectées par un rétrovirus humain de type HIV-2.

Un tel procédé peut être d'une mise en oeuvre particulièrement intéressante dans la mesure où il permettrait de détecter d'une façon précoce une infection due à un rétrovirus humain de type HIV-2 ou à un rétrovirus apparenté, dès lors que les inventeurs ont mis en évidence l'apparition de la gp300 à un stade précoce du cyle de réplication virale du rétrovirus responsable de l'infection.

Pour la mise en oeuvre du procédé de diagnostic précédent, les inventeurs ont conçu un nécessaire ou kit pour la détection d'anticorps dans les cellules d'un échantillon biologique d'un patient susceptible d'être infecté par un rétrovirus humain de type HIV-2, caractérisé en ce qu'il comprend :
- au moins une protéine et/ou une glycoprotéine antigénique précédemment décrite ou
- une composition élaborée à partir de ces composants, ou encore un mélange de ces différents constituants et
- des moyens permettant la réaction de formation du complexe immunologique entre les antigènes ci-dessus et les anticorps éventuellement présents dans l'échantillon biologique à tester, notamment si besoin un ou plusieurs tampons d'incubation,
- un échantillon de contrôle négatif,
- des moyens pour la révélation du complexe antigène-anticorps formé.

Ayant maintenant déterminé les différentes étapes de la formation du rétrovirus infectieux HIV-2, ainsi que l'importance de certaines d'entre elles dans le développement de l'infection, les inventeurs ont suggéré l'élaboration d'une composition pharmaceutique caractérisée en ce qu'elle comprend au moins un inhibiteur des glucosidases I et/ou II, notamment la castanospermine ou la deoxyjirimicyne, ou un mélange de ces inhibiteurs en une dose suffisante pour bloquer l'élimination des résidus glucose terminaux des chaînes oligosaccharidiques de la glycoprotéine gP150 lors du cycle viral, en association avec un excipient physiologiquement acceptable.

D'autres caractéristiques et avantages de l'invention apparaîtront à la lecture des exemples qui suivent ainsi que dans les figures dont la légende est donnée ci-après :

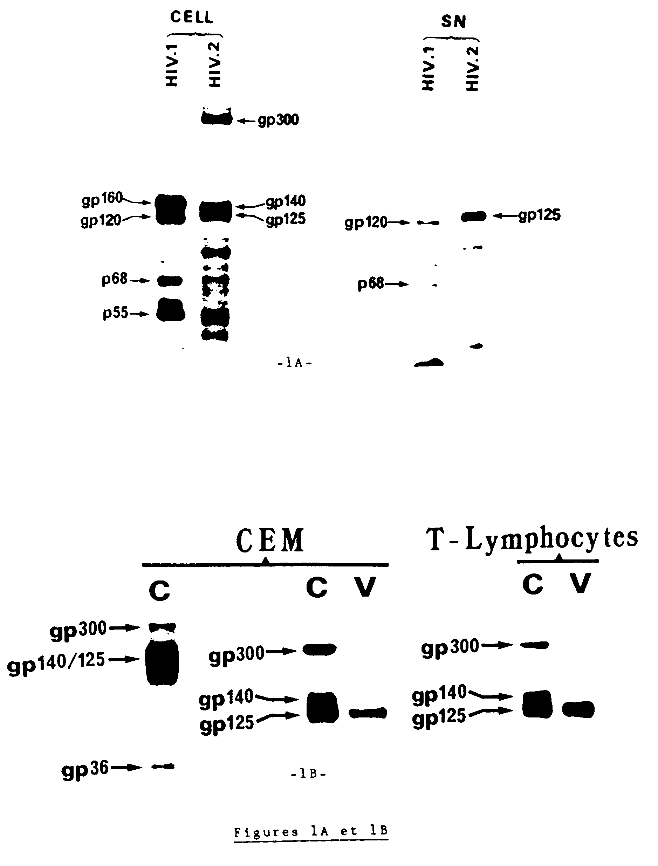

La figure 1 composée des figures 1A et 1B représente les glycoprotéines d'enveloppe de HIV-2 :

### - Figure 1A : Comparaison des protéines de haut poids moléculaire de HIV-1 et HIV-2.

Des cellules CEM infectées avec HIV-1 ou HIV-2 ont été marquées avec la ³⁵S-méthionine (200»Ci/ml ; 4 x 10⁶ cellules/ml) pendant 18 heures. Des extraits de cellules infectées (CELL) et leur milieu de culture correspondant (SN) ont été purifiés sur des colonnes d'immunoaffinité spécifiques : colonne HIV-1 sérum-Sépharose, spécifique des protéines de HIV-1 (Krust et al.,1988) et colonne HIV-2 sérum-Sépharose, spécifique des protéines de HIV-2 (voir "procédures expérimentales"). Les protéines purifiées ont été analysées par électrophorèse sur gel de polyacrylamide-SDS 7,5% contenant de l'urée 6M. Un fluorographe du gel est présenté sur la figure 1A. Les tailles des protéines de HIV-1 et de HIV-2 sont indiquées sur la gauche et sur la droite des lignes. La p68 et la p55 sont respectivement les transcriptase inverse et le précurseur de gag. La gp160 et la gp120 sont respectivement la glycoprotéine précurseur de l'enveloppe de HIV-1 et son produit de clivage. La gp300, la gp140 et la gp125 sont les précurseurs de la glycoprotéine et le produit de clivage de la protéine d'enveloppe de HIV-2.

### - Figure 1 B : Identification des glycoprotéines de HIV-2:

Des cellules CEM infectées par HIV-2 et des lymphocytes T ont été marquées avec de la ³H-glucosamine (200 »Ci/ml ; 4 X 10⁶ cellules/ml) pendant 18 heures. Des extraits de cellules infectées (ligne C) et le milieu de culture contenant le virus (ligne V) ont été purifiés sur colonne HIV-2 sérum-Sépharose et les protéines marquées ont été analysées par electrophorèse des glycoprotéines de HIV-2 synthétisées dans les cellules CEM sur gel 7,5%. Dans la ligne la plus à gauche, l'électrophorèse sur gel d'acrylamide 12,5% montre la présence de la gp36. Cette partie de la figure a dû être surexposée pour mettre en évidence la gp36, c'est pourquoi la gp140/125 est résolue sous forme d'une bande épaisse.

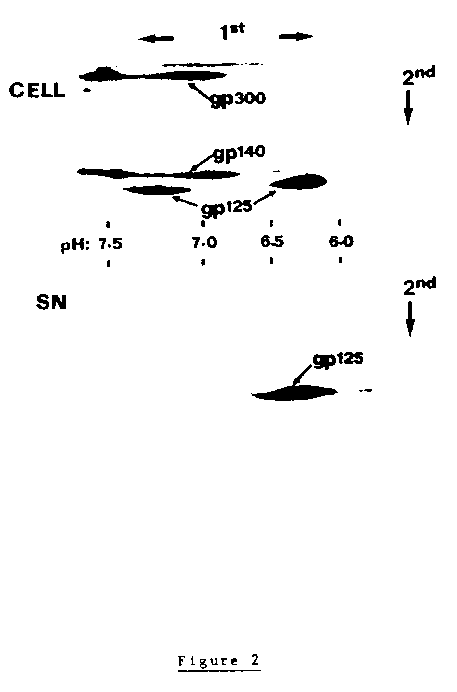

### - Figure 2 : Analyse de l'electrophorèse sur gel en deux dimensions, des glycoprotéines de HIV-2.

Les glycoprotéines gp300, gp140 et gp125 purifiées à partir de cellules CEM infectées par HIV-2 (CELL) ont été marquées avec la ³⁵S-méthionine. Les glycoprotéines, ainsi que le milieu de culture contenant le virus, ont été analysés (SN représente le résultat obtenu avec le milieu de culture ; les conditions de préparation sont semblables à celles de la figure 1A pour l'électrophorèse sur gel en deux dimensions (voir "procédures expérimentales"). Le gradient de pH de la focalisation isoélectrique (première dimension) correspond à celui qui a été donné. En seconde dimension, les protéines ont été résolues sur gel de polyacrylamide-SDS à 7,5% contenant de l'urée 6M. Des fluorographes des gels sont représentés sur la figure 2.

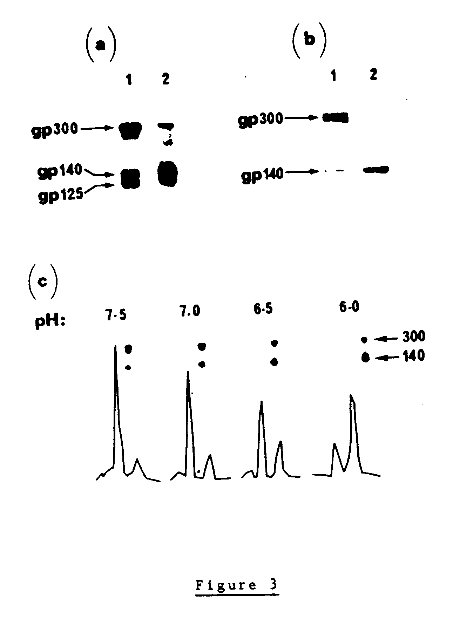

### - Figure 3 : Dissociation de la protéine gp300 native (a) et dénaturée (b et c).

a/ - Des extraits de cellules CEM infectées par HIV-2 et marquées à la ³⁵S-méthionine ont été purifiés sur colonne HIV-2 sérum-Sépharose. Cet échantillon a ensuite été divisé en deux parties aliquotes égales :
   l'une a été incubée dans un tampon d'accrochage (ligne 1) alors que l'autre a été incubée dans un tampon contenant 30mM d'acétate de sodium à pH4,0, 0,2mM PMSF, 100 unités/ml d'aprotinine et 5mM de β-mercaptoéthanol (ligne 2). Après 1 heure à 37°C, le milieu acide a été neutralisé et les deux échantillons ont été analysés par électrophorèse.
b/ - La gp300 marquée à la ³⁵S-méthionine, purifiée et lyophilisée a été suspendue dans un tampon d'acétate de sodium (100 »l) à pH 4 comme indiqué plus haut (ligne 2). Des incubations ont été conduites pendant 30 minutes à 37°C avant l'addition à deux reprises d'un tampon d'électrophorèse contenant de l'urée 2M. Les résultats de la ligne 1, correspondent à la gp300 lyophylisée directement suspendue dans le tampon d'électrophorèse.
c/ - La gp300 marquée par la ³⁵S-méthionine, purifiée et lyophilisée a été suspendue dans une solution contenant 30mM Tris-HCl, 0,2mM PMSF et 100 unités/ml d'aprotinine et tamponnée avec HCl à pH 7,5 ; 7,0 ; 6,5 ; et 6,0 (comme indiqué sur la figure). Après 60 minutes à 37°C on a ajouté à deux reprises un tampon d'électrophorèse et les échantillons ont été analysés par électrophorèse.

Des fluorographes de ces gels sont montrés en a, b et c. Dans la section c, la bande caractéristique de la gp300 est dissociée en gp140 cette dissociation pouvant être quantifiée par réalisation d'un scanner densitométrique de ce fluorographe.

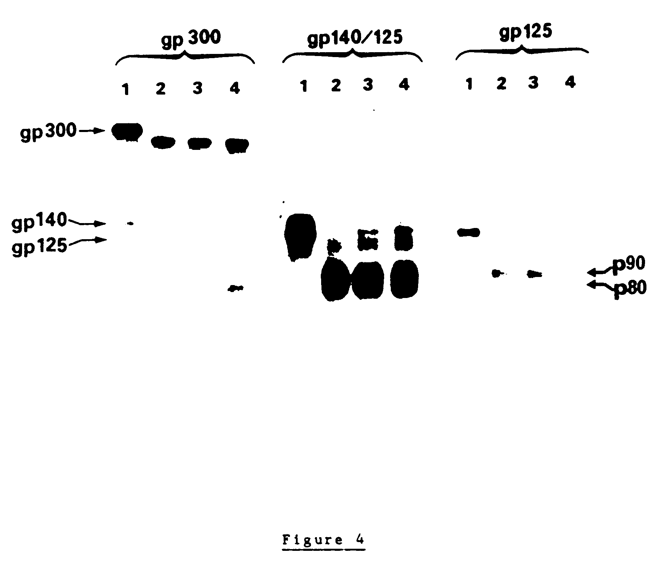

### - Figure 4 : Digestion des glvcoprotéines de HIV-2 avec Endo-H.

Les glycoprotéines gp300, gp140/gp125 et gp125, purifiées et lyophylisées (voir "procédures expérimentales") ont été suspendues dans un tampon contenant du citrate de sodium 150mM à pH 5,5 ; 0,1% SDS (w/v), 0,5mM PMSF avant d'être chauffées pendant 2 minutes à 90°C. Des parties aliquotes de ces échantillons ont été incubées (2 heures 30°C) sans endo-H (ligne 1) ou avec 0,4 (ligne 2), 2 (ligne 3) et 10 (ligne 4) milli-unités de Endo-H.

Toutes ces réactions ont été arrêtées par addition de tampon d'électrophorèse à deux reprises. L'électrophorèse était réalisée comme décrit pour la figure 1. Des fluorographes des différents gels ont été présentés. Les flèches p90 et p80 sur la droite indiquent la position du produit de digestion. Les conditions pour la digestion endo-H ont été décrites par Tarentino et al. 1974.

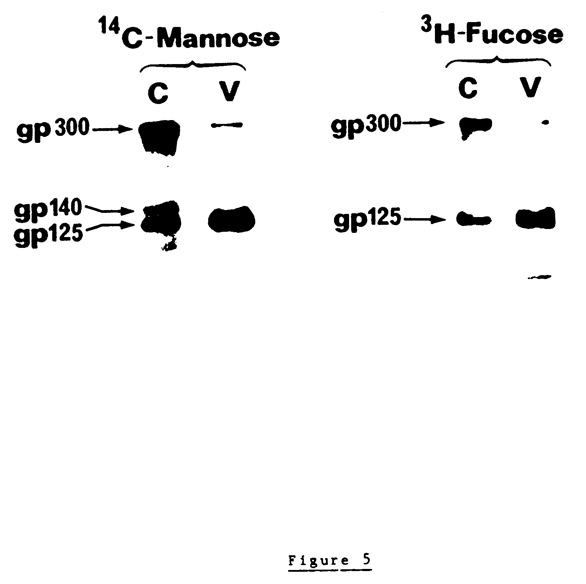

### - Figure 5 : Marquage isotopique des glycoprotéines de HIV-2 avec le 14^{C}-mannose ou le ³H-fucose.

Des cellules infectées par HIV-2 ont été marquées pendant 18 heures avec du 14^{C}-mannose (25 »Ci/ml ; 4 X 10⁶ cellules/ml) ou ³H-fucose (200 »Ci/ml ; 4 X 10⁶ cellules/ml). Des extraits des cellules infectées (ligne C) et du milieu de culture contenant le virus (ligne V) ont été purifiés sur colonne HIV-2 sérum-Sépharose. Les glycoprotéines marquées ont été ensuite analysées par électrophorèse sur gel de polyacrylamide. Le fluorographe est présenté à la figure 5.

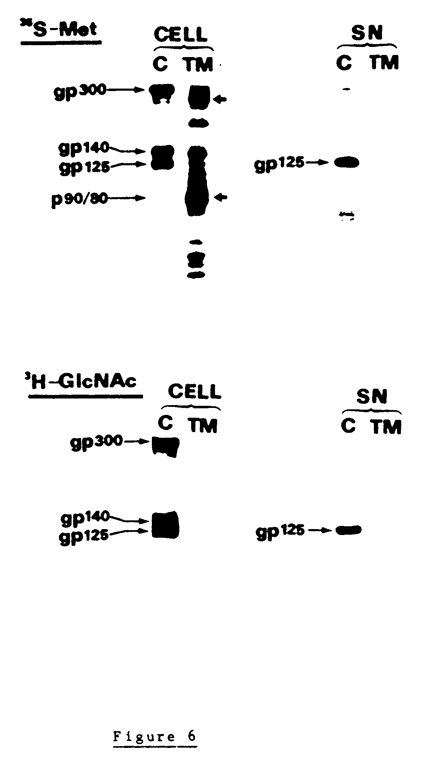

### - Figure 6 : Inhibition de la glycosylation liée à l'azote par la tunicamycine.

Des cellules infectées par HIV-2 en l'absence de tunicamycine (ligne C) ou en présence (ligne TM) de tunicamycine (2 »g/ml) ont été marquées avec la ³⁵S-méthionine (cadre intitulé ³⁵S-met ; 200 »Ci/ml ; 4 X 10⁶ cellules/ml) ou avec la ³H-glucosamine (cadre intitulé ³H-GLcNAc ; 200 »Ci/ml ; 4 X 10⁶ cellules/ml) pendant 16 heures. Les cellules traitées avec la tunicamycine ont tout d'abord été incubées (37°C) avec l'antibiotique (2 »g/ml) pendant 2 heures avant d'être marquées avec la ³⁵S-méthionine ou la 3H-glucosamine. Des extraits de cellules infectées (CELL) et du milieu de culture contenant le virus (SN) ont été purifiés sur colonne HIV-2 sérum-Sépharose et analysés par électrophorèse sur gel de poiyacrylamide 7,5%. Des fluorographes de ces gels sont présentés. Les positions du précurseur non glycosylé de l'enveloppe (p90/80) et du dimère non glycosylé (200Kd) sont indiquées par de petites fléches sur la droite. Les protéines 90/80Kd et 200Kd n'incorporent pas la ³H-glucosamine (cadre ³H-GlcNAc, CELL ligne TM).

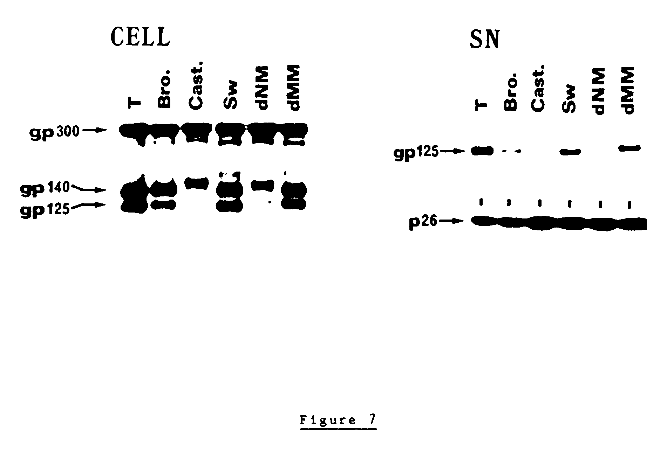

### - Figure 7 : Effet des inhibiteurs de coupures d'oligosaccharides sur le processus de formation de la glycoprotéine d'enveloppe de HIV-2.

Des cellules CEM infectées par HIV-2 ont été marquées (16 heures, 37°C) avec la ³⁵S-méthionine (200 »Ci/ml ; 4 X 10⁶ cellules/ml) en l'absence d'inhibiteur de coupure (lignes T) ou en présence d'un inhibiteur de coupure des oligosaccharides 1mM bromoconduritol (lignes Bro) ; 1mM castanospermine (lignes Cast.) ; 10 »g/ml swainsonine (lignes Sw) ; 3mM deoxynojirimycine (lignes dNM) et 1mM deoxymannojirimycine (lignes dMM). Des extraits des cellules infectées (cadre intitulé CELL) et du milieu de culture contenant les particules virales (cadre SN) ont été purifiés sur colonne HIV-2 sérum-Sépharose pour identifier les glycoprotéines virales gp125, gp140, gp300 dans les cellules infectées et gp125 dans les milieux de culture. Tous les échantillons ont été analysés par électrophorèse sur gel de polyacrylamide 7,5%. Afin de montrer que l'inhibition de la production de la gp125 par les cellules traitées avec différents inhibiteurs est spécifique de la glycoprotéine virale, le milieu de culture a été testé, conduisant à la détection de la présence de la protéine du noyau, p26, par un test d'immunoprécipitation faisant intervenir un sérum séropositif pour HIV-2 (Clavel et al.,1986a, 1987). La p26 a été analysée par électrophorèse sur gel de polyacrylamide (12,5%). Les figures représentent le fluorographe montrant seulement une seule partie de chaque gel.

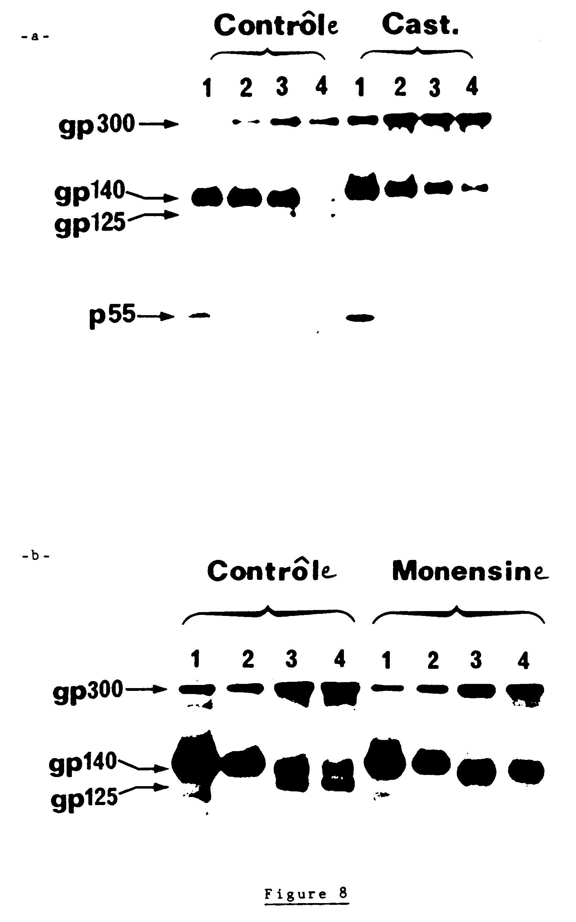

### - Figure 8 : Effet de la castanospermine et de la monensine sur le processus de formation des glycoprotéines de HIV-2.

a/ - Des expériences de marquage par un traceur radioactif pendant un temps court suivi d'une élimination de ce traceur et de son remplacement par des molécules non radioactives (expérience de "pulse-chase") ont été conduites à partir de cellules CEM infectées par HIV-2 en l'absence de castanospermine (contrôle) ou en présence de castanospermine 1mM (Cast.).
   Contrôle : Les cellules infectées ont été incubées pendant 1 heure à 37°C dans un milieu dépourvu de méthionine avant un marquage pendant une période brève de 15 minutes avec la ³⁵S-méthionine (200 »Ci/ml ; 4 X 10⁵ cellules/ml ligne 1). Le traceur radioactif éliminé et remplacé par le milieu de culture contenant de la methionine froide 5mM pendant 0,5 ; 1,5 et 3 heures (résultat rapporté dans les lignes 2, 3 et 4, respectivement). Cast. : Les cellules CEM infectées par HIV-2 ont été incubées (une heure à 37°C) dans un milieu dépourvu de méthionine contenant de la castanospermine, avant un marquage rapide pendant 30 minutes avec de la ³⁵S-méthionine). Ces cellules ont ensuite été suivies après élimination du traceur radioactif et remplacement par des molécules non radioactives comme décrit plus haut, mais en présence de castanospermine pendant 0,5 ; 1,5 et 3 heures (lignes 2, 3 et 4, respectivement).
b/ - Des expériences de marquage par un traceur radioactif et suivi de ce traceur dans les conditions décrites ci-dessus dans les cellules infectées par HIV-2 en l'absence de monensine (contrôle) ou en présence de monensine 1 »M ont été réalisées. Des cellules infectées avec ou sans la monensine ont été incubées (1 heure, 37°C) dans un milieu dépourvu de méthionine avant d'être marquées pendant une période brève (30 minutes) avec la ³⁵S-méthionine (ligne 1). Les cellules marquées ont été ensuite suivies dans le milieu de culture contenant de la méthionine froide 5mM pendant 0,5 ; 1,5 et 3 heures (lignes 2, 3 et 4 respectivement).

Des extraits ont été purifiés sur colonne HIV-2 sérum-Sépharose et les protéines marquées ont été analysées par électrophorèse sur gel de polyacrylamide 7,5%. Les résultats sont présentés sur des fluorographes. La p55 représente le précurseur de gag dans la section A, ligne 1.

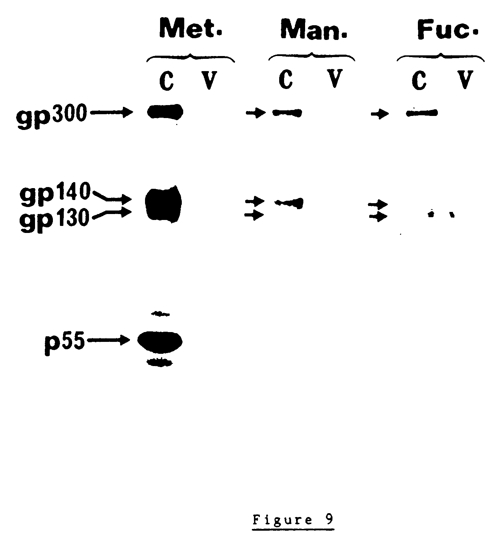

### - Figure 9 : Précurseurs des olygoprotéines de SIV.

Des cellules HUT-78 infectées par SIV ont été marquées (16 heures, 37°C) avec la ³⁵S-méthionine (200 »Ci/»l ; 4 X 10⁶ cellules/ml), ³H-fucose (200 »Ci/»l ; 4 X 10⁶ cellules/ml), et ¹⁴C-mannose (25 »Ci/»l; 4 X 10⁶ cellules/ml). Des extraits de cellules infectées (lignes C) et de milieu de culture contenant SIV (lignes V) ont été purifiés sur colonne de HIV-2 sérum-Sépharose. Un sérum positif pour HIV-2 a été utilisé pour effectuer la réaction d'immunoprécipitation des protéines de SIV, puisque les protéines de HIV-2 et de SIV présentent des réactions immunologiques croisées. Tous les échantillons ont été analysés par electrophorèse sur gel de polyacrylamide (7,5%) (voir "Procédures expérimentales"). Un fluorographe des différents gels est présenté. La mobilité électrophorétique de la gp300_{SIV} et de la gp140_{SIV} correspond à celle des glycoprotéines gp300 et gp140 de HIV-2. La gp130_{SIV} a une mobilité légèrement supérieure à celle de la gp125 de HIV-2. La p55 marquée avec la ³⁵S-méthionine est probablement le précurseur de gag de SIV.

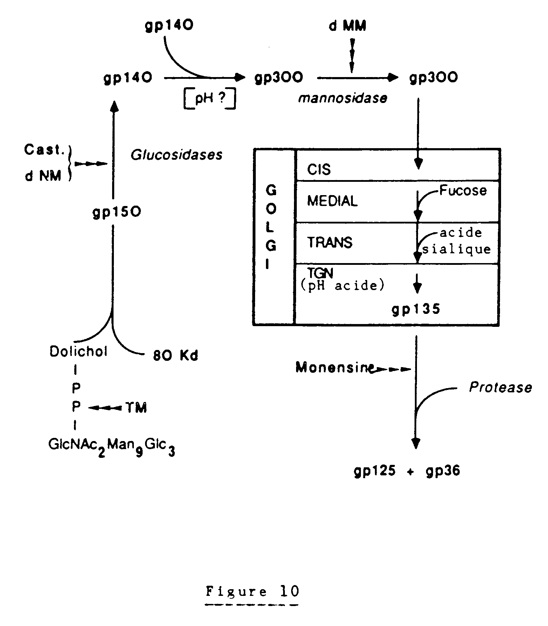

### - Figure 10 : Représentation schématique des étapes de formation de la glycoprotéine d'enveloppe de HIV-2.

Le polypeptide de 80Kd synthétisé et glycosylé donne lieu à la formation d'un "précurseur immature de glycoprotéines" gp150 qui est rapidement déglycosylé par les glucosidases du réticulum endoplasmique granuleux (RER). Une transformation de l'environnement (notamment du pH) entraîne alors la dimérisation donnant lieu à la formation de la gp300, "précurseur intermédiaire sous forme de dimère". La gp300 est coupée par les mannosidases du RER avant d'être transportée dans l'appareil de Golgi. D'autres coupures de la gp300 sont effectuées par les mannosidases du Golgi avant le transfert de fucose et de résidus d'acides sialiques dans le Golgi-médial et dans le trans-Golgi. Enfin, le dimère est dissocié en un "précurseur mature" gp135, en raison du pH acide règnant dans le compartiment du réseau trans-Golgi (TGN). La gp135 est transportée dans la membrane plasmique et clivée par la protéase cellulaire pour conduire à des glycoprotéines d'enveloppe matures de HIV-2, gp125 et gp36. La Tunicamycine (TM) inhibe l'assemblage des résidus dolichol-P-P-glycane ; la castanospermine (Cast.) et la déoxynojirimycine (dNM) inhibent les glucosidases du RER ; la déoxymannojurimycine (dMM) inhibe les mannosidases de Coupures dans le RER et dans le cis-Golgi et dans le Golgi-médial ; la monensine inhibe le transport des glycoprotéines de membrane et des protéines de sécrition à partir de l'appareil de Golgi.

### EXEMPLES

### PROCEDURES EXPERIMENTALES

### I - MATERIELS

L-(³⁵S)Methionine (activité spécifique) 1000 Ci/mmol, L-(6-³H) Fucose (activité spécifique : 45-70 Ci/mmol), D-(6-³H)Glucosamine (activité spécifique : 20-40 Ci/mmol) et D-(U-¹⁴C)Mannose (activité spécifique: 200-300 mCi/mmol) obtenus de Amersham (Amersham UK).

Le bromoconduritol, la castanospermine, la 1-deoxymannojirimycine (dMM) la 1-deoxynojirimycyne (dNM), la swainsonine et la tunicamycine ont été obtenus de Boehringer-Mannheim (Mannheim, RFA).

L'endoβ-N-acetylglucosaminidase H a été obtenue chez Calbiochem (San Diego, USA).

Les Ampholines proviennent de Pharmacia (Uppsala, Suède).

### II - VIRUS ET CELLULES

Un isolat HIV-1_{BRU} du virus d'immunodéficience humaine de type 1 (décrit par Montagniez et al 1984), un isolat de HIV-2_{ROD} du virus d'immunodéficience humaine du type 2 (décrit par Clavel et al. 1986a) et un virus d'immunodéficience simien SIVmac₁₄₂ (décrit par Daniel et al. 1985) ont été utilisés dans cette étude.

Les différentes lignées cellulaires et les lymphocytes humains ont été cultivés dans un milieu de suspension RPMI-1640 (GIBCO-BRL, Cergy-Pontoise France) contenant 10% (v/v) de sérum de veau foetal ; 2»g/ml de polybrene (SIGMA) ont été ajoutés aux cultures cellulaires infectées par HIV. Les cellules du clone CEM13 dérivent de la lignée CEM de cellules lymphoïdes humaines (déposée à l'ATCC sous le n° CCL119) et expriment l'antigène T4 à un haut niveau. 5 jours après l'infection avec l'isolat HIV-1_{BRU} ou avec l'isolat HIV-2_{ROD}, environ 80-90% des cellules produisent des particules virales et peuvent être identifiées par des effets cytopathogènes correspondant à la vacuolisation des cellules et à l'apparition de syncitia de petite taille.

La lignée cellulaire HUT-78 est une autre lignée cellulaire de lymphocytes T4 humains positifs (Gadzdar et al. 1980), hautement permissive pour la réplication de SIVmac₁₄₂ (Daniel et al. 1985). Des lymphocytes périphériques du sang de donneurs de sang sains ont été stimulés pendant 3 jours avec 0,02% (w/v) de fraction P de phytohémagglutinine (DIFCO, Detroit, USA) dans un milieu RPMI-1640, complété avec 10% de sérum de veau foetal. Les cellules ont ensuite été cultivées sur un milieu RPMI-1640 contenant 10% (v/v) de facteur de croissance de cellules T (TCGF,Biotest). Après l'infection avec HIV-2, des lymphocytes ont été cultivés en présence de 10% (v/v) TCGF et 2»g/ml de Polybrene.

### III - MARQUAGE METABOLIQUE DES CELLULES.

Pour le marquage métabolique des protéines, des cellules infectées ont été incubées pendant 16 heures à 37°C dans un milieu de culture MEM (abréviation anglaise de Minimum Essentiel Medium) sans L-methionine ni sérum, mais complété par 200»Ci/ml de L(³⁵S) méthionine. Pour le marquage métabolique des glycoprotéines, des cellules infectées ont été incubées pendant 16 heures à 37°C dans un milieu de culture MEM sans sérum ni glucose, mais complété par 200»Ci/ml H³-fucose et 200»Ci/ml de D-[6-H]-glucosamine ou 25»Ci/ml de ¹⁴C-mannose.

### IV - CELLULES ET EXTRAITS VIRAUX

Des culots cellulaires correspondant à 10⁷ cellules ont été resuspendus dans 100»l du tampon suivant : 10mM Tris-HCl pH 7,6, 150mM NaCl, 1mM EDTA, 0.2mM PMSF, 100 unités par ml d'aprotinine (Iniprol®, CHOAY) avant l'addition de 100»l du même tampon contenant 2% (v/v) de Triton®X-100. Des extraits cellulaires ont été centrifugés à 12.000g pendant 10 minutes et le surnageant a été conservé à -80°C jusqu'à utilisation. Pour les préparations d'extrait viral, 100»l de 10X tampon de lyse (100mM Tris-HCl pH 7,6, 1,5M NaCl, 10mM EDTA, 10% (v/v) Triton®X-100, 100 unités/ml aprotinine) ont été ajoutés par ml de surnageant clarifié de cellules CEM infectées. Ces Préparations ont été utilisées comme suit.

### V - PREPARATION D'UN IMMUNOABSORBANT AVEC DES ANTICORPS PRESENTS DANS LE SERUM D'UN PATIENT SEROPOSITIF POUR LE VIRUS HIV-2.

Des immunoglobulines d'un sérum d'un patient séropostifif pour HIV-2 ont été précipitées avec 50% de (NH₄)₂SO₄, dissous dans 20mM de phosphate de sodium (pH 8,0) puis purifiées sur une colonne de cellulose DEAE (DE 52,Whatman) par élution avec 20mM de phosphate de sodium (pH 8,0). Ces immunoglobulines ainsi purifiées ont été jugées pures à 90%. Les anticorps ont été ensuite couplés sur SEPHAROSE® CL 4B activé par CNBr conformément à la technique décrite par Berg (1977). Deux milligrames d'IgG ont été couplés par ml de SEPHAROSE® CL 4B. Cet immunoabsorbant est désigné sous la référence "HIV-2 sérum Sépharose".

### VI - LIAISONS DES PROTEINES HIV-2 SUR UNE COLONNE D'IMMUNOAFFINITE.

Des extraits cellulaires de cellules CEM produisant HIV-2 ont tout d'abord été dilués dans deux volumes de tampon d'accrochage (20mM Tris-HCl pH 7,6, 50mM KCl, 150mM NaCl, 1mM EDTA, 1% (v/v) Triton X-100®, 20% (v/v) glycérol, 7mM mercaptoethanol, 0,2mM PMSF, 100 unités/ml d'aprotinine) avant incubation avec un volume de "HIV-2 sérum-Sépharose". Des surnageants de cellules produisant le virus HIV-2 ont été traités de la même façon que les extraits cellulaires excepté qu'1/10ème de tampon d'accrochage concentré 10X a été ajouté par volume de surnageant. L'étape d'accrochage a été conduite pendant 1 nuit, puis la colonne a éte lavée suffisamment dans le tampon d'accrochage. Les protéines liées sur la colonne ont été éluées en les faisant bouillir dans un tampon d'électrophorèse (125mM Tris-HCl pH 6,8, 1% (w/v) SDS, 2M urea, 20% glycerol, 1% β-mercaptoethanol). Les protéines éluées ont été analysées sur gels d'electrophorèse SDS-polyacrylamide 7,5% contenant de l'urée 6M et 0,1% de bis-acrylamide au lieu de 0,2% (w/v).

### VII - ELECTROPHORESE PREPARATIVE.

Les glycoprotéines de HIV-2 éluées à partir de la colonne d'affinité ont été analysées par électrophorèse sur gel de polyacrylamide comme décrit précédemment et les régions du gel contenant les glycoprotéines virales ont été coupées par référence à la position des marqueurs protéiniques de poids moléculaire pré-établi (BRL).

Les glycoprotéines ont été éluées par incubation pendant 16 heures à 4°C dans un tampon d'élution (0,1M NaHCO₃, 0,5mM EDTA, 0,05% (w/v) SDS, 0,2mM PMSF). Les fractions de glycoprotéines ainsi obtenues ont été lyophilisées et gardées au réfrigérateur jusqu'à leur utilisation.

### VIII - ELECTROPHORESE SUR GEL EN DEUX DIMENSIONS.

Une électrophorèse sur gel en deux dimensions a été réalisée de la façon décrite par O'Farrel (1975) avec les modifications suivantes : des protéines marquées par la L(³⁵S)-méthionine liées sur une colonne de "HIV-2 sérum-Sépharose" ont été éluées en les faisant bouillir dans un tampon d'électrophorèse tel que décrit précedemment, avant dilution dans un volume de tampon contenant 9,5M d'urée, 8% (v/v)-mercaptoéthanol, 1,6% (w/v) d'ampholines à un pH situé dans l'intervalle 6,5-9, 0,4% (w/v) d'ampholines à un pH situé dans l'intervalle 3-10 et 100 unités/ml d'aprotinine.

### EXEMPLE 1 - CARACTERISATION DES GLYCOPROTEINES gp300 et gp140.

Le schéma de résolution obtenu à partir de l'analyse électrophorétique sur gel en 2 dimensions des glycoprotéines gp300, gp140 et gp125, marqué à la S³⁵-méthionine, montre que la gp300 et la gp140 sont apparentées. Ces deux protéines présentent des dépôts distincts sur le gel mais elles possèdent un point isoélectrique (pI) identique pour un pH variant de 6,8 à 7,8. Ces résultats sont rapportés sur la figure 2. Cette similitude entre les protéines gp140 et gp300 suggère que la gp300 est une forme dimérique de la gp140.

La gp125 présente moins d'hétérogénéité à la fois dans les cellules infectées et dans les particules virales et migre à un point isoélectrique par des valeurs de pH situées entre 6,2 et 6,5.

Dans les cellules infectées on a remarqué la présence de sous-entités mineures de la protéine gp125 ayant un point isoélectrique pour des valeurs de pH situées entre 7,2 et 7,3. Cette dernière catégorie de protéines n'est pas présente dans le virion HIV-2, c'est pourquoi elle représente vraisemblablement une glycoprotéine non définitivement constituée.

La nature acide de la gp125 mature peut être due à l'acide sialique au niveau de certaines chaînes carbohydrates ajoutées lors de la maturation de la glycoprotéine d'enveloppe.

La glycoprotéine d'enveloppe gp300 est très stable puisqu'elle résiste à des détergents ioniques (1% SDS) et non-ionique (2% Triton X-100®), à l'urée (2-6M), à des sels forts (1M NaCl) et à des agents réducteurs (1% β-mercaptoethanol). Cependant, on a pu établir que la gp300 peut être dissociée dans un milieu à pH acide en formant deux glycoprotéines gp140. Dans ces expériences, des protéines liées sur une colonne d'immunoaffinité ont eté incubées dans un tampon d'acétate à des valeurs de pH variant entre 4 et 7. Ces échantillons ont ensuite été analysés par électrophorèse sur gel de polyacrylamide. La figure 3A sur laquelle sont rapportés les résultats montre qu'une bande de gp300 est convertie en une bande correspondant à la position de la gp140 lorsque l'échantillon est incubé à pH4. D'autres expériences ont été mises en oeuvre en utilisant des préparations purifiées de gp300 obtenues à partir des gels d'électrophorèse préparative comme précédemment décrit. De tels échantillons dénaturés de la gp300 ont été dissociés complètement dans un tampon acétate à pH 6,0. Les résultats de ces essais sont rapportés sur la figure 3B. L'efficacité de la dissociation de la glycoprotéine gp300 purifiée est vraisemblablement due à l'abaissement du pH et à la présence de résidus SDS dans l'échantillon lyophilisé, étant donné que la glycoprotéine gp300 native liée sur colonne n'était pas dissociable dans le même tampon à des valeurs de pH plus élevées que pH5.

Dans un tampon Tris-HCl la dissociation est moins efficace. A pH 7,5 on remarque seulement une dissociation faible de la gp300 sous forme de gp140, ceci augmentant toutefois avec l'abaissement des valeurs du pH. Dans un tampon Tris à pH 6,0, la dissociation était d'environ 80% comme le montre la figure 3C. Lors de la dissociation de la gp300 pure, dans un autre tampon acétate ou Tris-HCl aucune autre protéine que la gp140 n'a pu être détectée (les expériences ont été conduites sur gel de polyacrylamide 15%). Ces résultats indiquent que la gp300 est une forme dimérique de la gp140, elle-même précurseur de la glycoprotéine d'enveloppe de HIV-2. En conséquence, il apparaît vraisemblable que lors de la constitution de la glycoprotéine d'enveloppe, deux molécules de gp140 participent à un mécanisme de fusion dépendant du pH.

### EXEMPLE 2 - CARACTERISATION DES CHAINES LATERALES D'OLIGOSACCHARIDES DES GLYCOPROTEINES DE HIV-2.

Une digestion avec l'endo β-N-acetylglucosaminidase H (endo H) a permis de mettre en évidence pour les glycoprotéines de HIV-2 la présence d'oligosaccharides liés sur l'azote (Tarentino et al 1974). La gp300, le mélange de (gp140, gp125) et la gp125 seule ont été purifiés par chromatographie d'affinité et électrophorèse préparative (voir les procédures expérimentales). Les échantillons lyophilisés ont été suspendus dans un tampon de digestion endo H qui ne favorise pas la dissociation de gp300 en gp140.

Après digestion avec l'endo H, la mobilité électrophorétique de la gp300 correspond à celle plus faible d'une protéine de 200-250Kd. Une petite fraction de gp300 dissociée en gp140 a été digérée en donnant lieu à une protéine de 80Kd. Les résultats sont rapportés à la figure 4, section gp300. L'échantillon contenant le mélange de gp140, gp125 a été digéré par endo H en donnant des protéines de 90 et 80Kd, alors que la gp125 seule était convertie en une protéine de 90Kd lors de cette digestion comme le montrent les sections gp140/125 et gp125 de la figure 4. Une digestion avec l'endo H de la gp140 et de la gp125 conduit à des produits de poids moléculaires 80 et 90Kd respectivement.

En effet, en marquant métaboliquement les cellules avec du ¹⁴C-mannose et du ³H-fucose on a montré l'incorporation de mannose dans la gp300, la gp140 et la gp125 alors que seules la gp300 et la gp125 étaient capables d'incorporer du fucose (figure 5). Les résidus fucose sont normalement transférés sur des chaînes d'oligosaccharides lors d'un stade avancé du cycle de glycosylation, après l'action d'enzymes de cohésion du réticulum endoplasmique et de l'appareil de Golgi (Kornfeld et Kornfeld, 1985 ; Fuhrmann et al 1985).

Pour ces raisons on peut penser que selon toute vraisemblance, la faible résistance à la digestion avec l'endo H de la gp125 par rapport à la gp140 est due à la conversion de certaines chaînes latérales d'oligosaccharides du type mannose en des oligosaccharides complexes lors de la transformation de la glycoprotéine d'enveloppe (Kornfeld et Kornfeld, 1985). Le fait que la gp140 ne contienne aucun résidu fucose est en accord avec le fait qu'elle constitue un précurseur de la gp300 et de la gp125.

### EXEMPLE 3 - EFFET DE LA TUNICAMYCINE, INHIBITEUR DE LA GLYCOSYLATION SUR LA FORMATION DES GLYCOPROTEINES DE HIV-2.

Toutes les glycoprotéines porteuses d'oligosaccharides liés sur l'azote reçoivent leur chaîne oligosaccharidique Glc₃Man₉-GlcNAc₂-pp-Dolichol, grâce à une réaction réalisée par la protéine-oligosaccharidyl transférase qui catalyse le transfert en bloc des chaînes d'oligosaccharides sur des résidus d'asparagine (voir les références Kornfeld et Kornfeld 1985). La Tunicamycine bloque une telle glycosylation liée à l'azote en inhibant la production de N-acétyiglucosamine pyrophosphoryldolichol, première étape dans l'assemblage des oligosaccharides liés par les lipides (Li et al. 1978 ; Heifetz et al. 1979). En présence de 2 »g/ml de tunicamycine, la totalité de la glycosylation liée sur l'azote, des glycoprotéines d'enveloppe de HIV-2 dans les cellules infectées est bloquée. Ce résultat est démontré par l'absence d'incorporation de ³H-glucosamine dans les glycoprotéines virales gp300, gp140, gp125 (figure 6). Dans ces conditions expérimentales, la synthèse de protéine n'a toutefois pas été affectée dans les cellules infectées, traitées par de la tunicamycine. De telles cultures marquées avec des isotopes de la ³⁵S-méthionine présentent deux protéines majeures de poids apparent 200 et 80-90Kd, migrant en de larges bandes (figure 6). Le poids moléculaire de ces protéines coïncide avec les produits de la digestion par l'endo H des gp300, gp140 et gp125 (figure 4), suggèrant que les protéines de poids moléculaire 200Kd, 80-90Kd correspondent à des formes non glycosylées des glycoprotéines d'enveloppe de HIV-2.

Le poids moléculaire de la protéine de 80-90Kd correspond au poids moléculaire attendu du précurseur de l'enveloppe de HIV-2 non glycosylé, estimé à partir de sa séquence d'acide nucléique (Guyader et al. 1987). La glycoprotéine de 200Kd est probablement la forme dimérique du précurseur d'enveloppe non glycosylé. Ces résultats confirment que les protéines d'enveloppe de HIV-2 contiennent des chaînes polysaccharidiques liées à l'azote. Outre l'inhibition de la glycosylation, un traitement à la tunicamycine inhibe la formation et l'exportation de la glycoprotéine d'enveloppe puisque la protéine de 80-90Kd n'a pas été mise en évidence dans le milieu extra-cellulaire, comme le montre la figure 6, ligne SN.

Les résultats de ces expériences nous permettent de supposer que les chaînes de glycosaccharides des protéines d'enveloppe de HIV-2 sont vraisemblablement impliquées dans le transport cellulaire à travers l'appareil de Golgi. L'absence de formes non glycosylées de la protéine d'enveloppe dans le milieu extra-cellulaire de cellules traitées par la tunicamycine pourrait éventuellement être due à sa dégradation rapide. De nombreux rapports ont suggéré que de façon générale, la forme non-glycosylée d'une protéine est plus sensible à l'action des protéases que sa forme glycosylée (Olden et al. 1978 ; Schwartz et al. 1976). En accord avec ces résultats, les protéines de petit poids moléculaire présentes dans les cellules marquées par la ³⁵S-méthionine cultivées dans la tunicamycine représentent probablement des produits de dégradation partielle de la protéine d'enveloppe non glycosylée (figure 6).

### EXEMPLE 4 - EFFETS DES INHIBITEURS DE COHESION D'OLIGOSACCHARIDE SUR LA SYNTHESE ET LA TRANSFORMATION DE GLYCOPROTEINES DE HIV-2.

Des oligosaccharides liés par l'asparagine (Glc₃Man₉GlcNAC₂) des glycoprotéines subissent des modifications extensives ou une tranformation à la suite de leur attachement aux protéines (voir Kornfeld et Kornfeld, 1985). Les réactions d'élimination des extrémités glucose et mannose par les enzymes ont lieu dans le lumen du réticulum endoplasmique granuleux (RER) et dans l'appareil de Golgi par action de mannosidases et de glucosidases spécifiques.

On peut intervenir sur la transformation des chaînes d'oligosaccharides des glycoprotéines en utilisant des inhibiteurs spécifiques des glucosidases et des mannosidases (rapporté par Schwarz et Datema, 1984 ; Fuhrmann et al. 1985). Dans ces expériences, on a utilisé différents inhibiteurs de jonction pour investiger la localisation des précurseurs de la glycoprotéine de HIV-2 et aussi pour étudier le rôle de la glycosylation dans la transformation du précurseur de l'enveloppe. Les inhibiteurs utilisés sont les suivants la castanospermine, alcaloïde de plante qui inhibe la glucosidase I (Saul et al 1983) ; la déoxynojirimycine (dNM), analogue de glucose qui inhibe les glucosidases I et II assurant l'élimination des extrémités glucose (Lemansky et ai. 1984) : un déoxymannojirimycine (dMM), analogue de mannose qui inhibe les réactions catalysées par la mannosidase (Fuhrmann et al, 1984) ; le bromoconduritol (6-bromo-3,4,5-trihydroxycyclohex-1-ene) qui inhibe la glucosidase II (Datema et al, 1982) ; la swainsonine, alcaloïde indolizidine qui inhibe la mannosidase II de l'appareil de Golgi (Tulsiani et al. 1982).

Les cellules infectées par HIV-2 marquées par des isotopes de la ³⁵S-méthionine en l'absence ou en présence de différents inhibiteurs de jonction et de protéines intra-cellulaires et extra-cellulaires ont été purifiées par chromatographie d'affinité (figure 7). On a vérifié que les cellules infectées contiennent la gp300, la gp140 et la gp125, alors que seule la gp125 est observée dans le milieu extra-cellulaire (les résultats sont rapportés sur la figure 7, sections "cellules" et SN, lignes T). Dans les cellules traitées avec la castanospermine ou le dNM, on a noté un taux normal de gp300, l'absence de gp125 et une petite quantité d'une protéine de 150Kd qui correspond probabblement à la forme glycosylée de la gp140. En conséquence, dans de telles cellules la transformation de la glycoprotéine d'enveloppe a été bloquée puisqu'aucune gp125 n'est détectée dans le milieu extra-cellulaire malgré la production de p26, protéine du noyau de HIV-2 (figure 7, lignes Cast. et dNM).

Ces résultats montrent gue l'enlèvement des résidus glucose terminal des chaînes d'oligosaccharides du précurseur de la glycoprotéine d'enveloppe est nécessaire pour sa transformation et le clivage par la protéase cellulaire. Le bromoconduritol qui agit sur la glycosidase II, inhibait aussi à 70-90% la production normale de gp125, mais les taux de gp140 et gp300 restaient normaux (figure 7, lignes Bro.). Au contraire de la castanospermine et du dNM (qui inhibent l'enlèvement du résidu glucose terminal), le traitement au bromoconduritol (qui inhibe l'enlèvement des deux résidus glucose internes) ne bloque pas complètement la transformation de la glycoprotéine d'enveloppe de HIV-2. En fait, un taux faible de gp125 peut être détecté dans les milieux intra-cellulaires et extra-cellulaires. Ce dernier résultat suggère qu'un faible niveau d'élimination de mannose peut avoir lieu sans l'enlèvement des deux résidus glucose internes.

Des inhibiteurs de mannosidase, la swainsonine et le dMM n'ont pas engendré de modifications apparentes dans le taux intra-cellulaire de gp300, de gp140 ou de gp125, mais le niveau extra-cellulaire de gp125 était de 50% inférieur à la forme correspondante dans les cellules de contrôle (figure 7, lignes Sw et dMM). Ainsi bien que la chaîne d'oligosaccharides ait seulement subi une déglycosylation le précurseur de la glycoprotéine est clivé grâce à des enzymes protéolytiques produisant une protéine similaire de la gp125, mais contenant plus de mannose, ce qui affecte probablement le transport cellulaire de la gp125. Le poids moléculaire de la glycoprotéine extra-cellulaire produite en présence de dMM est légèrement plus élevé que celui de la protéine produite en l'absence de l'inhibiteur. Ceci est vraisemblablement dû à une concentration supérieure en résidus mannose de la protéine extra-cellulaire synthétisée par les cellules traitées au dMM (figure 7, section SN).

On peut en déduire que les effets des inhibiteurs d'enzymes d'élimination sur la transformation de la glycoprotéine d'enveloppe de HIV-2 sont spécifiques puisque la synthèse et la production de la p26 de HIV-2 ne sont pas affectées du tout (figure 7, section SN).

### EXEMPLE 5 - EFFET DE LA CASTANOSPERMINE ET DE LA MONENSINE SUR LA TRANSFORMATION DES GLYCOPROTEINES OE HIV-2.

Afin d'étudier la transformation intra-cellulaire des glycoprotéines de HIV-2, des expériences mettant en oeuvre des traceurs (pulse-chase) ont été réalisées. Les cellules infectées par HIV-2 ont été rapidement marquées par la ³⁵S-méthionine radioactive pendant 15 minutes puis l'incorporation du traceur a été suivie (chase) pendant 0.5, 1.5 et 3 heures (figure 8, contrôle). La gp140 est la première protéine détectée 15 minutes après le marquage. Pendant le suivi de l'incorporation du traceur radioactif la gp300 peut être détectée après une demi-heure, alors que la gp125 n'est détectée qu'aprés 1,5 à 3 heures. Le fait que la gp300 est observée après la synthèse de la gp140, et que la gp125 n'est détectée qu'après formation de la gp300 (figure 8A, lignes 1-4) suggère que la dimérisation est un stade intermédiaire nécessaire à la transformation de l'oligosaccharide en une glycoprotéine parvenue à maturation, la gp125. Cette suggestion est confirmée par l'utilisation de castanospermine, qui inhibe la suppression du résidu glucose externe et des chaînes polysaccharidiques. Après 30 minutes de marquage (pulse), en présence de castanospermine, une protéine de 150Kd est détectée avec la gp300 (figure 8, Cas., ligne 1). La protéine de 150Kd pourrait correspondre à la gp140 ; le faible accroissement du poids moléculaire du premier précurseur est attribué à la présence de résidus glucose dans les chaînes d'oligosaccharides. Ainsi, la gp140 synthétisée dans des cellules infectées par HIV-2, représente le précurseur de la glycoprotéine sans les résidus glucose. En accord avec ce résultat, la protéine de 150Kd (gp150) représente la première glycoprotéine de l'enveloppe de HIV-2 non encore parvenue à maturation. L'enlèvement des résidus glucose dans les cellules de contrôle correspond à une transformation rapide ayant lieu pendant ou immédiatement après la translocation cotranslationelle des précurseurs de glycoprotéines dans le réticulum endoplasmique (Lemansly et al, 1984). Aprés 30 minutes de marquage (pulse), et 3 heures de suivi de l'incorporation du traceur (chase), en présente de castanospermine, le niveau de gp150 est graduellement réduit alors que la quantité gp300 croît (figure 8, Cast. lignes 1-4). Dans ces conditions expérimentales, le précurseur n'a pas été clivé en donnant la gp125.

Une autre caractérisation de la glycoprotéine d'enveloppe de HIV-2 a été réalisée lors d'expériences relatives à l'incorporation de traceur radioactif (pulse chase), en utilisant la monensine, ionophore cationique qui inhibe le transport des protéines de l'appareil de Golgi vers la membrane plasmique ou dans certains cas, bloque le transport de protéines au niveau du cisternae de l'appareil de Golgi médian (Tartakoff et Vassali, 1977 ; Johnson et Schlesinger, 1980 ; Strous et Lodish, 1980 ; Griffiths et al. 1983). Des cellules infectées par HIV-2 d'une part en l'absence, d'autre part en présence de monensine ont été marquées avec un traceur radioactif (pulse) pendant 30 minutes puis l'incorporation du traceur a été suivie (chase) pendant 0,5, 1,5 et 3 heures (figure 8B). En présence de monensine, les cellules infectées par HIV-2 ont synthétisé la gp140 à un niveau normal, ainsi que sa forme dimérique. A l'inverse aucune trace de gp125 n'a pu être détectée dans les cellules traitées par la monensine. Après 1,5-3 heures de suivi de l'incorporation du traceur (chase), les cellules traitées par la monensine ont accumulé une protéine de 135Kd (gp135) qui est probablement le produit de dissociation du précurseur du dimère. Le poids moléculaire légèrement plus faible de la gp135 par rapport à la gp140 est dû à l'enlèvement de certains résidus mannose par l'action des mannosidases du RER et de l'appareil de Golgi. Cette gp135 peut être ensuite transportée dans la membrane plasmatique et clivée par la protéase cellulaire. L'inhibition du transport de protéines par la monensine, bloque la glycoprotéine gp135 dans la partie "trans" de l'appareil de Golgi (trans Golgi). Aucune glycoprotéine d'enveloppe à maturation n'est détectée dans les cellules traitées par la monensine, que ce soit intra-cellulairement ou extra-cellulairement, alors que la protéine du noyau (core) p.26 est synthétisée et excrétée.

### EXEMPLE 6 - DIMERISATION DU PRECURSEUR DE GLYCOPROTEINES DANS LES CELLULES INFECTEES PAR SIVmac.

La séquence nucléotidique de la glycoprotéine d'enveloppe de HIV-2 montre une homologie considérable (75% d'acides aminés identiques) avec celle de SIV (Guyader et al. 1987 ; Chakrabarti et al. 1987 ; Franchini et al. 1987). Pour ces raisons, des investigations ont été faites afin de déterminer si la dimérisation des glycoprotéines d'enveloppe peut être également détectée dans des cellules infectées par SIV. Des protéines de SIV ont été purifiées sur colonne d'immunoaffinité contenant des anticorps spécifiques dirigés contre les protéines de HIV-2 dans la mesure où les protéines gag, pol et env de HIV-2 et de SIV, présentent des réactions antigéniques croisées. La figure 9 montre que les cellules infectées par SIV synthétisent trois protéines de haut poids moléculaire, analogues à celles synthétisées dans les cellules infectées par HIV-2 : gp300, gp140 et gp130. La preuve que les glycoprotéines présentes dans les cellules infectées par SIV sont des glycoprotéines a été donnée par le marquage isotypique avec le ¹⁴C-mannose et le ³H-fucose. Ces trois protéines ont incorporé du mannose mais seules les gp300/SIV et la gp130/SIV ont incorporé le fucose. La gp300/SIV et la gp140/SIV sont des protéines intra-cellulaires alors que la gp 130/SIV est une glycoprotéine extra-cellulaire. Le fait que la gp300/SIV et la gp130/SIV sont capables d'incorporer le fucose montre que ces protéines sont des produits de transformation de la gp140/SIV.

Ces résultats indiquent qu'une formation en doublet (dimère) du précurseur de la glycoprotéine d'enveloppe est une propriété spécifique de l'expression du gène de l'enveloppe de HIV-2 et de SIV. On peut a l'inverse soutenir que la glycoprotéine d'enveloppe de HIV-1 ne subit pas de dimérisation pendant sa transformation. Les cellules infectées par HIV-1 en présence de castanospermine ou de dNM n'accumulent pas de dimère d'enveloppe, comme c'est le cas pour HIV-2 ou SIV.

### DISCUSSION :

Comme le montrent les exemples, les inventeurs sont parvenus à déterminer le processus de formation de la glycoprotéine d'enveloppe de HIV-2 et ils ont mis en évidence un mécanisme original dans la synthèse des glycoprotéines comprenant des chaînes oligosaccharidiques liées sur l'azote. Les glycoprotéines d'enveloppe de HIV-2, à savoir la glycoprotéine gp125 extracellulaire, et la glycoprotéine gp36 transmembranaire, dérivent d'une glycoprotéine précurseur commune (Guyader et al., 1987). Un élément inattendu rapporté dans l'invention est que, pour être transporté et transformé dans l'appareil de Golgi, le précurseur de la glycoprotéine implique la formation d'un dimère homologue. Le mécanisme de dimérisation de la glycoprotéine d'enveloppe n'est pas encore totalement clair. Le fait que le dimère purifié dans le cadre de l'invention puisse être dissocié à un pH acide (pH 6,0) suggère que la dimérisation pourrait être dépendante du pH.

Les inventeurs ont mis en évidence le fait que les chaînes d'oligosaccharides présentes dans le précurseur naturel de la glycoprotéine ne sont pas essentielles pour la formation du dimère. Ceci a été démontré lors de deux expériences : (1) la digestion avec l'endo H conduit à une modification de la mobilité électrophorétique du dimère sans dissociation de celui-ci ; (2) en présence de tunicamycine, les cellules infectées par HIV-2 synthétisent un précurseur d'enveloppe non glycosylé (80-90Kd) et capable de former un dimère (200Kd). Ces résultats attestent le fait que la formation d'un dimère est une propriété intrinsèque de l'ossature polypeptidique du précurseur de l'enveloppe.

Des expériences de marquage pendant une période courte (en anglais "pulse") avec un traceur radioactif, puis d'une étape d'élimination par lavage de ce traceur et de son remplacement par des molécules non radioactives et le suivi de la localisation de la radioactivité (en anglais "chase") à partir d'échantillons prélevés à différents intervalles de temps, avec ou sans castanospermine (figure 8) montrent que la dimérisation du précurseur de la glycoprotéine intervient normalement immédiatement après la suppression des résidus glucose. Les glucosidases étant associées aux membranes du réticulum endoplasmique, on peut supposer que la dimérisation intervient dans le RER. En présence de castanospermine, le dimère est accumulé dans le RER et n'est pas transformé. Cependant, lorsque les résidus glucose sont supprimés, l'inhibition des mannosidases du RER n'empêche pas la transformation des glycoprotéines dimèrisées à travers l'appareil de Golgi (figure 7). Ceci permet de faire l'hypothèse que les résidus glucose des chaînes oligosaccharidiques du précurseur dimérisé empêchent son expulsion du RER. En accord avec ceci, l'hypothèse peut être émise que la coupure des résidus glucose est nécessaire pour un transport efficace du RER vers le Golgi, ceci étant éventuellement dû au fait que les oligosaccharides déglucosylés forment une partie du site de reconnaissance pour un récepteur du transport (Lodish et Kong, 1984, Lemansky et al.,1984). Il est également possible que la suppression du glucose soit importante pour que le précurseur dimérisé prenne une configuration fonctionnelle correcte (Schlesinger et al., 1984). Cette configuration fonctionnelle favoriserait l'action des mannosidases de coupure.

Au vu de ces résultats, les inventeurs ont proposé un schéma de représentantion de la transformation des glycoprotéines d'enveloppe de HIV-2 (figure 10). La taille présumée de l'ossature polypeptidique du précurseur de la glycoprotéine d'enveloppe est d'environ 80Kd (figures 4 et 6). La chaîne oligosaccharidique est transférée du dolichol-P-P vers un précurseur d'enveloppe (80Kd) prosaslement sur des résidus amino-acides de type asparagine fonctionnant comme accepteurs (Kornfeld et Kornfeld 1985). La tunicamycine inhibe l'assemblage du dolichol-P-P et du glycane, et pour cette raison la protéine de 80Kd n'est pas glycosylée. L'addition de chaînes oligosaccharidiques à la protéine de 80Kd conduit à l'obtention du premier précurseur de la glycoprotéine d'enveloppe, la gp150. Ce précurseur pourrait exister tel quel ou au contraire ne pas exister dans les cellules infectées, car l'addition de chaînes polysaccharidiques et la coupure des résidus glucose a lieu prosaslement pendant la traduction du précurseur. Quoiqu'il en soit, la gp150 est rapidement déglucosylée pour aboutir à la gp140. A ce stade une différence dans l'environnement, peut être au niveau du pH, guiderait la formation du dimère par fusion de 2 molécules de gp140. La gp300 résultant, peut alors être coupée par une mannosidase du RER, et transportée vers l'appareil de Golgi. En présence de castanospermine ou de dNM, la gp150 est dimérisée et accumulée dans le RER. Ce dimère n'est pas transformé parce qu'il est glucosylé. Cependant, aussi longtemps que le dimère est présent dans une forme déglucosylée, il peut être transporté dans le Golgi ; l'inhibition de la mannosidase du RER par le dMM ne bloque pas l'obtention du précurseur dimérisé.

Dans le Golgi, la gp300 traverse les différents compartiments probablement par transport vésiculaire (Griffiths et Simons, 1986). Pendant ce transport la chaîne oligosaccharidique est coupée par les mannosidases du Golgi avant l'addition d'autres sucres tels que le fucose et l'acide sialique. La démonstration de l'incorporation du fucose a été obtenue par marquage isotypique de la gp300 avec le ³H-fucose. La démonstration de l'incorporation de l'acide sialique a été obtenue indirectement par digestion de la gp300 avec la neuramidase, enzyme capable d'hydrolyser les acides N-acetylneuraminiques, terminaux dans différentes glycoprotéines (Peyrieras et al. 1983). La gp300 de HIV-2 est susceptible d'être digérée avec la neuramidase, comme le montre la diminution significative de la mobilité électrophorétique du dimère. Les résultats sont en accord avec l'observation que le précurseur garde sa forme dimérisée tout au long de son transport et de sa transformation dans le Golgi cis, médial et trans cisternae avant d'être transporté dans le réseau du trans-Golgi (TGN ; Griffiths et Simons, 1986) où il est dissocié lors d'un abaissement du pH de ce compartiment. Le dimère dissocié conduit à des glycoprotéines (gp135) d'un poids moléculaire légèrement inférieur à celui du précurseur de la glycoprotéine le premier détecté (gp150-140). La gp135 pourrait être transportée vers la membrane plasmique et être ainsi clivée par la protéase cellulaire avant de conduire à des glycoprotéines matures de l'enveloppe de HIV-2, gp125 et gp36. La monensine inhibe probablement le transport du Golgi vers la membrane plasmique; pour cette raison la gp135 est accumulée dans le Golgi.

Il est connu que l'appareil de Golgi est impliqué dans le mécanisme du tri des protéines de secrétion et des protéines de la membrane plasmique. Ce mécanisme semble avoir lieu dans le compartiment terminal du Golgi désigné par TGN (Griffiths et Simons, 1986). Ce compartiment correspond au côté "trans" des empilements de Golgi désigné parfois par lysosomes du réticulum endoplasmique de Golgi (GERL) et désigné récemment par post vacuoles du Golgi ou cisternae le plus interne du complexe de Golgi (Novikoff, 1976 ; Saraste et Kuismanen 1984 ; Orci et al. 1987). De façon tout à fait intéressante on a étudié le pH du TGN et on a remarqué qu'il était moyennement acide, c'est-à-dire d'environ 6 (Anderson et Pathak, 1985 ; Griffiths et Simons, 1986). Le pH acide dans le TGN pourrait prendre part dans la dissociation du dimère formé.

Les résultats discutés précédemment illustrent le fait que la formation et la transformation des glycoprotéines d'enveloppe de HIV-2 entrent dans un processus comprenant plusieurs étapes et impliquant notamment la synthèse d'un précurseur immature gp150-140, la synthèse d'un précurseur dimérisé intermédaire ou gp300 et, finalement d'un précurseur mature la gp135. En dépit de leur parenté les rétrovirus HIV-1 et HIV-2 ont des mécanismes différents pour la formation de leurs glycoprotéines d'enveloppe, ce qui peut être une caractéristique tout à fait avantageuse pour distinguer entre une infection due à HIV-1 et une infection due à HIV-2.

## Revendications

1. Glycoprotéine rétrovirale antigénique caractérisée en ce qu'il s'agit :
- soit d'une glycoprotéine, appelée gp300, ayant un poids moléculaire (PM) d'environ 300Kd, et constituée par l'association de deux unités d'une glycoprotéine gp140 précurseur de la glycoprotéine d'enveloppe gp125 codée par le gène env du génome d'un rétrovirus humain du type HIV-2 susceptible de provoquer un SLA ou SIDA,
- soit du dimère du précurseur de la glycoprotéine d'enveloppe d'un rétrovirus qui croise immunologiquement avec des anticorps dirigés contre ladite glycoprotéine gp300.

2. Glycoprotéine antigénique gp300 selon la revendication 1, caractérisée par les propriétés suivantes :
- elle est présente dans les cellules d'un échantillon biologique d'un malade infecté par un rétrovirus humain du type HIV-2, notamment dans les lymphocytes T4 humains ou dans des lignées permanentes dérivées de ces lymphocytes T4,
- son point isolélectrique (pI) est obtenu par un pH appartenant à l'intervalle [6,8 - 7,8],
- elle est glycosylée par des chaînes oligosaccharidiques liées à l'azote,
- elle est stable dans les détergents ioniques, notamment le SDS 1%, dans les détergents non-ioniques, notamment le Triton ®X-100 2%, dans l'urée 4M, dans les sels forts et les agents réducteurs,
- elle se dissocie spontanément in vitro en deux glycoprotéines d'un poids moléculaire d'environ 140Kd, entre pH 4-5,
- après isolement et purification sur gel d'électrophorèse, elle se dissocie, dans un tampon acétate, in vitro en deux glycoprotéines d'un poids moléculaire d'environ 140Kd, pour des valeurs du pH égales ou inférieures à 6,
- elle présente une bande d'électrophorèse sur gel de polycrylamide SDS 7,5% en présence d'urée 6M, correspondant à un poids moléculaire de 300Kd après purification sur colonne d'immunoaffinité du type HIV-2 sérum-Sépharose^{R}, contenant des anticorps spécifiques obtenus à partir du sérum d'un patient infecté par un rétrovirus humain du type HIV-2.

3. Glycoprotéine antigénique selon la revendication 1 ou la revendication 2, caractérisée en ce qu'elle n'est pas révélée par analyse en électrophorèse d'un milieu biologique constitué de particules virales présentes dans le milieu de culture spécifique de cellules infectées par un rétrovirus humain du type HIV-2 après marquage, avec de la ³⁵S-méthionine (200»Ci/ml. 4 x 10⁶ cellules/ml) pendant 18 heures et purification des extraits obtenus contenant les cellules infectées et leur milieu de culture correspondant alors qu'elle est révélée dans les mêmes conditions dans les susdites cellules infectées.

4. Protéine p200 dérivée de la glycoprotéine selon l'une quelconque des revendications 1 à 3, caractérisée par une ossature non-glycosylée identique à celle de ladite glycoprotéine, ayant un poids moléculaire d'environ 200Kd et se distinguant de ladite glycoprotéine, par l'absence de groupes de glycosylation.

5. Glycoprotéine purifiée gp150 susceptible d'être détectée in vitro dans les cellules d'un patient infecté par un rétrovirus humain du type HIV-2,
caractérisée par :
- un poids moléculaire d'environ 150Kd,
- l'ossature peptidique du précurseur de la glycoprotéine d'enveloppe gp125, codée par le gène env du génome d'un rétrovirus humain du type HIV-2 ou d'un rétrovirus dont la glycoprotéine externe d'enveloppe est reconnue par les anticorps d'un sérum de patient infecté par ledit rétrovirus humain de type HIV-2, notamment les anticorps dirigés contre la gp125,
- des résidus glucose terminaux sur une chaîne d'oligosaccharides.

6. Composition antigénique pour le diagnostic de la présence d'anticorps dans les cellules d'un échantillon biologique d'un patient infecté par un rétrovirus humain du type HIV-2, caractérisée en ce qu'elle comprend une protéine ou une glycoprotéine antigénique selon l'une quelconque des revendications 1 à 5.

7. Anticorps monoclonal caractérisé en ce qu'il reconnaît la glycoprotéine selon l'une quelconque des revendications 1 à 3, en ce qu'il ne reconnaît pas les glycoprotéines d'enveloppe gp125 et gp36 matures.

8. Anticorps monoclonal caractérisé en ce qu'il reconnaît un épitope qui dans la glycoprotéine selon l'une quelconque des revendications 1 à 3 est exposé et accessible à l'anticorps, cet épitope étant cependant soit non-exposé soit masqué dans les gp125 et gp36 matures, dans leur conformation naturelle.

9. Anticorps selon la revendication 8, caractérisé en ce qu'il est en outre neutralisant notamment en ce qu'il empêche la fixation d'un rétrovirus humain du type HIV-2 sur son récepteur, en particulier sur le récepteur T4 des lymphocytes humains qui le comportent.

10. Anticorps monoclonal caractérisé en ce qu'il reconnaît la p200 selon la revendication 4, et en ce qu'il ne reconnaît pas les glycoprotéines gp125 et gp36 matures.

11. Anticorps monoclonal caractérisé en ce qu'il reconnaît un épitope de la p200 selon la revendication 4, cet épitope étant cependant soit non-exposé soit masqué sur le gp125 et la gp36 et/ou sur la gp300 telles qu'obtenues dans leur conformations naturelles.

12. Anticorps monoclonal selon la revendication 11, caractérisé en ce qu'il est neutralisant notamment en ce qu'il empêche la fixation d'un rétrovirus humain du type HIV-2 sur son récepteur notamment sur le récepteur T4 des lyphocytes humains.

13. Composition immunogène contenant une glycoprotéine selon l'une quelconque des revendications 1 à 3 ou la p200 selon la revendication 4, en association avec un véhicule pharmaceutiquement acceptable, approprié pour la préparation de vaccins.

14. Composition immunogène selon la revendication 11, caractérisée en ce qu'elle est dosée en protéine et en glycoprotéine antigénique selon l'une quelconque des revendications 1 à 4, de façon à permettre l'administration d'une dose de 10 à 100»g par kilogramme.

15. Procédé de préparation de la glycoprotéine antigénique selon l'une quelconque des revendications 1 à 3, caractérisé par les étapes suivantes :
- lyse de cellules infectées par un rétrovirus humain du type HIV-2 et séparation du surnageant et de l'extrait cellulaire,
- incubation de l'extrait cellulaire et/ou du surnageant sur une colonne d'immunoaffinité, sur laquelle est déposé un immunoabsorbant renfermant des anticorps purifiés, tels qu'obtenus à partir du sérum d'un malade infecté par un rétrovirus humain du type HIV-2, ces anticorps étant fixés avantageusement sur un support adapté, en présence d'un tampon et pendant un temps suffisamment long pour obtenir la formation d'un complexe immunologique antigène-anticorps,
- lavage de la colonne avec un tampon pour éliminer les molécules non retenues sur le support,
- résolution des glycoprotéines éluées,
- récupération des protéines antigéniques recherchées.

16. Procédé selon la revendication 15 pour la préparation de la glycoprotéine selon l'une quelconque des revendications 1 à 3, caractérisé en ce que la résolution des glycoprotéines est faite par électrophorèse et par électroélution des protéines retenues sur la colonne.

17. Procédé selon la revendication 15 pour la préparation de la glycoprotéine selon l'une quelconque des revendications 1 à 3, caractérisé en ce que la résolution des glycoprotéines est obtenue par
- élution des protéines fixées sur la colonne d'immunoaffinité ci-dessus,
- purification des produits ainsi éluées sur une colonne de chromatographie contenant, fixés sur le support de séparation des anticorps monoclonaux reconnaissant la gp300,

18. Procédé pour le diagnostic in vitro de la présence d'anticorps dans les cellules d'un échantillon biologique d'un patient en vue de vérifier une infection éventuelle par un rétrovirus humain du type HIV-2, caractérisé par :
- la mise en contact dans des conditions autorisant la formation d'un complexe immunologique du type antigène-anticorps, de cet échantillon biologique avec un antigène selon l'une quelconque des revendications 1 à 5, ou une composition antigénique selon la revendication 6,
- la détection du complexe antigène-anticorps formé.

19. Nécessaire ou kit pour la détection d'anticorps dans les cellules d'un échantillon biologique d'un patient susceptible d'être infecté par un rétrovirus humain du type HIV-2, caractérisé en ce qu'il comprend :
- au moins une protéine ou/et une glycprotéine antigénique selon l'une quelconque des revendications 1 à 5,
- ou une composition selon la revendication 6,
- ou encore un mélange de ces différents constituants et
- des moyens permettant la réaction de formation du complexe immunologique entre les antigènes ci-dessus et les anticorps éventuellement présents dans l'échantillon biologique à tester, notamment si besoin un ou plusieurs tampons d'incubation,
- un échantillon de contrôle négatif,
- des moyens pour la révélation du complexe antigène-anticorps formé.

## Claims

1. Antigenic retroviral glycoprotein characterized in that it is :
- either a glycoprotein called gp300, having a molecular weight of about 300 kD and constituted by the association of two units of a glycoprotein gp140, precursor of the envelope glycoprotein gp125 encoded by the env gene of a human HIV-2 type retrovirus capable of causing a LAS or AIDS,
- or the dimer of the precursor of the envelope glycoprotein of a retrovirus which cross-reads immunologically with antibodies directed against the said glycoprotein gp300.

2. Antigenic glycoprotein gp300 according to Claim 1, characterized by the following properties :
- it is present in the cells of a biological sample taken from a patient infected by a human HIV-2 type retrovirus, in particular in human T4 lymphocytes or in permanent lines derived from these T4 lymphocytes,
- its isoelectric point (pI) lies in the pH range 6.8 - 7.8,
- it is glycosylated by oligosaccharide chains linked to nitrogen,
- it is stable to ionic detergents, in particular 1% SDS, non-ionic detergents, in particular 2% Triton^{R} X-100, 4M urea, strong salts and reducing agents,
- it dissociates spontaneously in vitro into two glycoproteins of a molecular weight a about 140 kD between pH 4 - 5,
- after isolation and purification on gel electrophoresis, it dissociates in acetate buffer in vitro into two glycoproteins of molecular weight of about 140 kD at pH values equal to or less than 6,
- it exhibits one band on electrophoresis in a 7.5% polyacrylamide-SDS gel in the presence a 6M urea, corresponding to a molecular weight a 300 kD after purification on an immunoaffinity column of the Sepharose^{R} HIV-2 type serum containing specific antibodies obtained from the serum of a patient infected by a human HIV-2 type retrovirus.

3. Antigenic glycoprotein according to Claim 1 or Claim 2, characterized in that it is not revealed by electrophoretic analysis of a biological medium constituted by viral particles present in the culture medium specific for cells infected by a human HIV-2 type retrovirus after labelling with ³⁵S-methionine (200 »Ci/ml 4 x 10⁶ cells/ml) for 18 hours and purification of the extracts obtained containing the infected cells and their corresponding culture medium whereas it is revealed under the same conditions in the above-mentioned infected cells.

4. Protein p200 derived from the glycoprotein according to any one of the Claims 1 to 3, characterized by a non-glycosylated skeleton identical with that of the said glycoprotein having a molecular weight of about 200 kD and differing from the said glycoprotein by the absence of glycosyl groups.

5. Purified glycoprotein gp150 capable of being detected in vitro in the cells of a patient infected by a human HIV-2 type retrovirus, characterized by :
- a molecular weight of about 150 kD,
- the peptide skeleton of the preoursor of the envelope glycoprotein gp125 encoded in the env gene of the genome of a human HIV-2 type retrovirus or of a retrovirus whose external envelope protein is recognized by the antibodies of the serum of a patient infected by the said human HIV-2 type retrovirus, in particular the antibodies directed against gp125,
- terminal glucose residues on an oligosaccharide chain.

6. Antigenic composition for the diagnosis of the presence of antibodies in the cells of a biological sample taken from a patient infected by a human HIV-2 type retrovirus, characterized in that it comprises a protein or antigenic glycoprotein according to any one of the Claims 1 to 5.

7. Monoclonal antibody characterized in that it recognizes the glycoprotein according to any one of the Claims 1 to 3, and in that it does not recognize the mature envelope glycoproteins gp125 and gp36.

8. Monoclonal antibody characterized in that it recognizes an epitope which in the glycoprotein according to any one of the Claims 1 to 3 is exposed and accessible to the antibody, this epitope being however either not exposed or masked in the mature gp125 and gp36 in their natural conformation.

9. Antibody according to Claim 8, characterized in that it is in addition neutralizing in particular in that it prevents the binding of a human HIV-2 type retrovirus to its receptor, in particular to the T4 receptor of the human lymphocytes which bear it.

10. Monoclonal antibody characterized in that it recognizes p200 according to Claim 4, and in that it does not recognize the mature glycoproteins gp125 and gp36.

11. Monoclonal antibody characterized in that it recognizes an epitope of the p200 according to Claim 4, this epitope being however either not exposed or masked on the gp125 and the gp36 and/or on the gp300 in their natural conformations.

12. Monoclonal antibody according to Claim 11, characterized in that it is neutralizing, in particular in that it prevents the binding of a human HIV-2 type retrovirus to its receptor, in particular to the T4 receptor of human lymphocytes.

13. Immunogenic composition containing a glycopeptide according to any one of the Claims 1 to 3 or the p200 according to Claim 4, in combination with a pharmaceutically acceptable vehicle, suitable for the preparation of vaccines.

14. Immunogenic composition according to Claim 11, characterized in that it is dosed in protein and antigenic glycoprotein according to any one a the Claims 1 to 4 so as to permit the administration of a dose of 10 to 100 »g per kilogram.

15. Procedure for the preparation of the antigenic glycoprotein according to any one of the Claims 1 to 3 characterized by the following steps :
- lysis of cells infected by a human HIV-2 type retrovirus and separation of the supernatant from the cell extract,
- incubation of the cell extract and/or the supernatant on an immunoaffinity column on which is deposited an immuno-absorbent containing purified antibodies such as those obtained from the serum of a patient infected by a human HIV-2 type retrovirus, these antibodies being advantageously bound to a suitable support in the presence of a buffer and for a sufficiently long to lead to the formation of an antigen-antibody immunological complex,
- washing of the column with a buffer in order to remove the molecules not retained by the support,
- resolution of the glycoproteins eluted,
- recovery of the desired antigenic proteins.

16. Procedure according Claim 15 for the preparation of the glycoprotein according to any one of the Claims 1 to 3, characterized in that the resolution of the glycoproteins is performed by electrophoresis and by electroelution of the proteins retained on the column.

17. Procedure according to the Claim 15 for the preparation of the glycoprotein according to any one of the Claims 1 to 3, characterized in that the resolution of the glycoproteins is obtained by
- elution of the proteins bound to the above immunoaffinity column,
- purification of the products thus eluted on a chromatographic column containing as separation support monoclonal antibodies recognizing gp 300 bound.

18. Procedure for the in vitro diagnosis of the presence of antibodies in the cells of a biological sample taken from a patient for the purpose of verifying a possible infection by a human HIV-2 type retrovirus, characterized by:
- the placing of this biological sample in contact with an antigen according to any one of the Claims 1 to 5 or an antigenic composition according to Claim 6 under conditions permitting the formation of an antigen-antibody type immunological complex,
- the detection of the antigen-antibody complex formed.

19. Kit for the detection of antibodies in the cells of a biological sample of a patient likely to be infected by a human HIV-2 type retrovirus, characterized in that it contains :
- at least one protein and/or antigenic glycoprotein according to any one of the Claims 1 to 5,
- or a composition according to Claim 6,
- or even a mixture of these different constituents and
- agents permitting the formation of the immunological complex between the above antigens and the antibodies possibly present in the biological sample to be tested, in particular if necessary one or more incubation buffers,
- a negative control sample,
- agents for the revelation of the antigen-antibody complex formed.

## Patentansprüche

1. Retrovirales antigenes Glykoprotein, nämlich
- ein Glykoprotein mit der Bezeichnung gp300, mit einem Molekulargewicht (MG) von ungefähr 300 kD und bestehend aus der Kombination von zwei Einheiten eines Glykoprotein gp140-Vorläufers des Hüllglykoproteins gp125, das durch das env-Gen des Genoms eines menschlichen Retrovirus vom Typ HIV-2 codiert wird, das LAS oder AIDS verursachen kann, oder
- ein Dimer des Vorläufers des Hüllglykoproteins eines Retrovirus, das mit Antikörpern, die gegen das Glykoprotein gp300 gerichtet sind, immunologisch kreuzreagiert.

2. Antigenes Glykoprotein gp300 nach Anspruch 1, gekennzeichnet durch die folgenden Eigenschaften:
- es liegt in den Zellen einer biologischen Probe eines Patienten vor, der mit einem menschlichen Retrovirus vom Typ HIV-2 infizier ist, insbesondere in den menschlichen T4-Lymphocyten oder in den permanenten Linien, die von diesen T4-Lymphocyten stammen,
- sein isoelektrischer Punkt (pI) liegt in einem pH-Bereich von 6,8 - 7,8,
- es ist mit Oligosaccharidketten glykosyliert, die über den Stickstoff gebunden sind,
- es ist in ionischen Detergentien stabil, insbesondere in 1 % SDS, in nicht-ionischen Detergentien, insbesondere 2 % Triton®X-100, in 4 M Harnstoff, in Salzen und in Reduktionsmitteln,
- es dissoziiert in vitro spontan in zwei Glykoproteine mit Molekulargewichten von etwa 140 kD, in einem pH-Bereich zwischen 4 und 5,
- nach Isolierung und Reinigung durch Gelelektrophorese dissoziiert es in einem Acetatpuffer in vitro in zwei Glykoproteine mit Molekulargewichten von etwa 140 kD bei pH-Werten, die 6 entsprechen oder darunter liegen,
- es zeigt nach der Reinigung an einer Immunaffinitätssäule vom Typ HIV-2 Serum-Sepharose® mit spezifischen Antikörpern, die aus Serum eines Patienten erhalten wurden, der mit einem menschlichen Retrovirus vom Typ HIV-2 infiziert ist, auf einem 7,5%igen Polyacrylamid-SDS-Gel in Gegenwart von 6 M Harnstoff eine Bande, die einem Molekulargewicht von 300 kD entspricht.

3. Antigenes Glykoprotein nach Anspruch 1 oder 2, dadurch gekennzeichnet, daß es bei einer elektrophoretischen Analyse eines biologischen Mediums nicht gefunden wird, das aus viralen Partikeln besteht, die in dem spezifischen Kulturmedium von Zellen vorliegen, die durch ein menschliches Retrovirus vom Typ HIV-2 infiziert sind, nach Markierung mit ³⁵S-Methionin (200 »Ci/ml, 4 x 106 Zellen/ml) während 18 Stunden und Reinigung der erhaltenen Extrakte, die infizierte Zellen und deren entsprechendes Kulturmedium enthalten, während es unter den gleichen Bedingungen in den infizierten Zellen selbst gefunden wird.

4. Protein p200, das von dem Glykoprotein nach einem der Ansprüche 1 bis 3 stammt, gekennzeichnet durch ein nicht-glykosyliertes Grundgerüst, das mit dem des Glykoproteins identisch ist, mit einem Molekulargewicht von etwa 200 kD, und das sich von dem Glykoprotein durch die Abwesenheit von Glykosylierungsresten unterscheidet.

5. Gereinigtes Glykoprotein gp150, das in vitro in den Zellen eines Patienten nachgewiesen werden kann, der mit einem menschlichen Retrovirus des Typs HIV-2 infiziert ist, gekennzeichnet durch:
- Molekulargewicht von etwa 150 kD,
- das Peptidgerüst des Vorläufers des Hüllglykoproteins gp125, das von dem env-Gen des Genoms eines menschlichen Retrovirus vom Typ HIV-2 codiert wird oder von einem Retrovirus, dessen Glykoprotein der äußeren Hülle von Antikörpern aus dem Serum eines Patienten erkannt wird, der mit dem menschlichen Retrovirus vom Typ HIV-2 infiziert ist, insbesondere Antikörper, die gegen gp125 gerichtet sind,
- terminale Glucosereste auf einer Oligosaccharidkette.

6. Antigene Zusammensetzung für die Diagnose der Gegenwart von Antikörpern in den Zellen einer biologischen Probe eines Patienten, der mit einem menschlichen Retrovirus vom Typ HIV-2 infiziert ist, dadurch gekennzeichnet, daß sie ein Protein oder ein antigenes Glykoprotein nach einem der Ansprüche 1 bis 5 umfaßt.

7. Monoclonaler Antikörper, dadurch gekennzeichnet, daß er das Glykoprotein nach einem der Ansprüche 1 bis 3 erkennt, daß er aber die reifen Hüllglykoproteine gp125 und gp36 nicht erkennt.

8. Monoclonaler Antikörper, dadurch gekennzeichnet, daß er ein Epitop erkennt, das auf dem Glykoprotein nach einem der Ansprüche 1 bis 3 exponiert und für den Antikörper zugänglich ist, wobei das Epitop jedoch in den reifen Proteinen gp125 und gp36 in ihrer natürlichen Konformation entweder nicht exponiert oder maskiert ist.

9. Antikörper nach Anspruch 8, dadurch gekennzeichnet, daß er außerdem neutralisierend wirkt, insbesondere indem er die Bindung eines menschlichen Retrovirus vom Typ HIV-2 an seinen Rezeptor hemmt, insbesondere an den T4-Rezeptor menschlicher Lymphocyten, die ihn tragen.

10. Monoclonaler Antikörper, dadurch gekennzeichnet, daß er das Protein p200 nach Anspruch 4 erkennt und daß er die reifen Glykoproteine gp125 und gp36 nicht erkennt.

11. Monoclonaler Antikörper, dadurch gekennzeichnet, daß er ein Epitop des Proteins p200 nach Anspruch 4 erkennt, wobei das Epitop jedoch entweder nicht exponiert oder maskiert auf den Proteinen gp125 und gp36 und/oder auf dem Protein gp300 in ihrer natürlichen Konformation vorliegt.

12. Monoclonaler Antikörper nach Anspruch 11, dadurch gekennzeichnet, daß er neutralisierend wirkt, insbesondere, daß er die Bindung eines menschlichen Retrovirus vom Typ HIV-2 an seinen Rezeptor hemmt, insbesondere an den T4-Rezeptor menschlicher Lymphocyten.

13. Immunogene Zusammensetzung, enthaltend ein Glykoprotein nach einem der Ansprüche 1 bis 3 oder das Protein p200 nach Anspruch 4, in Verbindung mit einem pharmazeutisch verträglichen Vehikel geeignet für die Herstellung von Impfstoffen.

14. Immunogene Zusammensetzung nach Anspruch 13, dadurch gekennzeichnet, daß sie aus einem Protein und einem antigenen Glykoprotein nach einem der Ansprüche 1 bis 4 zusammengesetzt ist, so daß die Verabreichung einer Dosis von 10 bis 100 »g pro kg möglich ist.

15. Verfahren zur Herstellung des antigenen Glykoproteins nach einem der Ansprüche 1 bis 3, gekennzeichnet durch die folgenden Schritte:
- Lyse der Zellen, die mit einem menschlichen Retrovirus vom Typ HIV-2 infiziert sind, und Antrennung des Überstandes und des Zellextrakts,
- Inkubation des Zellextrakts und/oder des Überstandes auf einer Immunaffinitätssäule mit einem Immunabsorbens, das gereinigte Antikörper umfaßt, die vom Serum eines Patienten erhalten wurden, der mit einem menschlichen Retrovirus vom Typ HIV-2 infiziert ist, wobei diese Antikörper vorteilhafterweise an einen passenden Träger gebunden sind, in Gegenwart eines Puffers und für einen Zeitraum, der lang genug ist zur Bildung eines immunologischen Antigen-Antikörper-Komplexes,
- Waschen der Säule mit einem Puffer zur Entfernung der Moleküle, die nicht an den Träger gebunden werden,
- Auftrennung der eluierten Glykoproteine,
- Gewinnung der gesuchten antigenen Proteine.

16. Verfahren nach Anspruch 15 für die Herstellung des Glykoproteins nach einem der Ansprüche 1 bis 3, dadurch gekennzeichnet, daß die Auftrennung der Glykoproteine durch Elektrophorese und durch Elektroelution der Proteine erfolgt, die auf der Säule zurückgehalten werden.

17. Verfahren nach Anspruch 15 für die Herstellung des Glykoproteins nach einem der Ansprüche 1 bis 3, dadurch gekennzeichnet, daß die Auftrennung der Glykoproteine folgendermaßen durchgeführt wird:
- Elution der an die vorstehend erwähnte Immunaffinitätssäule gebundenen Proteine,
- Reinigung der so eluierten Produkte auf einer Chromatographiesäule, die, gebunden an das Trenngel, monoclonale Antikörper enthält, die gp300 erkennen.

18. Verfahren für die in vitro-Diagnose der Gegenwart von Antikörpern in den Zellen einer biologischen Probe eines Patienten, um eine eventuelle Infektion mit einem menschlichen Retrovirus vom Typ HIV-2 festzustellen, gekennzeichnet durch die folgenden Schritte:
- Inkontaktbringen der biologischen Probe mit einem Antigen nach einem der Ansprüche 1 bis 5 oder einer antigenen Zusammensetzung nach Anspruch 6 unter Bedingungen, die die Bildung eines immunologischen Antigen-Antikörper-Komplexes erlauben,
- Nachweis des gebildeten Antigen-Antikörper-Komplexes.

19. Kit zum Nachweis von Antikörpern in den Zellen einer biologischen Probe eines Patienten, der mit einem menschlichen Retrovirus vom Typ HIV-2 infiziert sein kann, gekennzeichnet durch folgende Bestandteile:
- mindestens ein Protein und/oder ein antigenes Glykoprotein nach einem der Ansprüche 1 bis 5,
- oder eine Zusammensetzung nach Anspruch 6,
- oder auch ein Gemisch dieser verschiedenen Bestandteile und
- Mittel, um die Reaktion zur Bildung des immunologischen Komplexes zwischen den vorstehenden Antigenen und den gegebenenfalls in der zu untersuchenden biologischen Probe vorhandenen Antikörpern zu ermöglichen, insbesondere, falls notwendig, mit einem oder mehreren Inkubationspuffern,
- eine Negativ-Kontrollprobe,
- Mittel zum Nachweis des gebildeten Antigen-Antikörper-Komplexes.
